# EUROPEAN PATENT SPECIFICATION

(11) **EP 3 582 794 B1**
(45) Date of publication and mention of the grant of the patent: **22.04.2026**
(21) Application number: 18754087.7
(22) Date of filing: 20.02.2018
(51) Int. Cl.: A61K 35/28, A61K 35/407, A61K 35/22, A61K 35/35, A61P 1/16, A61P 13/12

(54) **FAT-ASSOCIATED LYMPHOID CLUSTERS AS SITES FOR TRANSPLANTATION,TISSUE REGENERATION, ORGANOGENESIS AND FUNCTION FOR MULTIPLE TISSUES**
FETTASSOZIIERTE LYMPHOID-CLUSTER ALS STELLEN ZUR TRANSPLANTATION, GEWEBEREGENERATION, ORGANOGENESE UND FUNKTION FÜR MEHRERE GEWEBE
GROUPES LYMPHOÏDES ASSOCIÉS À LA GRAISSE COMME SITES DE TRANSPLANTATION, DE RÉGÉNÉRATION TISSULAIRE, D'ORGANOGENÈSE ET DE FONCTION POUR DE MULTIPLES TISSUS

(30) Priority: 17.02.2017 US 201762460267 P; 18.10.2017 US 201762574119 P
(43) Date of publication of application: 25.12.2019
(73) Proprietor: University of Pittsburgh- Of the Commonwealth System of Higher Education, Pittsburgh, PA 15260 (US)
(72) Inventor: LAGASSE, Eric, Pittsburgh PA 15215 (US)
(74) Representative: Barker Brettell LLP
(86) International application number: PCT/US2018/018684
(87) International publication number: WO 2018/152488

(56) References cited:
- WO-A1-2014/138486
- US-A1- 2011 002 899
- US-A1- 2016 058 794
- US-B2- 9 493 532
- BENEZECH ET AL.: "Inflammation-induced formation of fat-associated lymphoid clusters", NAT IMMUNOL, vol. 16, no. 8, August 2015 (2015-08-01), pages 819 - 828, XP055532649

## Description

### 1. INTRODUCTION

The present disclosure relates to the engraftment and proliferation of organ cells in fat-associated lymphoid clusters ("FALCs," a.k.a. "milky spots") to generate ectopic tissue that may be used to supplement or replace organ function in a subject.

### 2. BACKGROUND OF THE INVENTION

There is a dearth of effective treatments for patients with liver disease. Liver diseases are responsible for over 31,000 deaths annually in the United States. Orthotopic liver transplantation (OLT) is too often the last resort and currently the only curative treatment for severe disease (1, 2). Moreover, although an estimated 100,000 patients are in need of a new liver, only just over 6,000 patients receive a liver transplant each year. The shortage of available donors is one of the major challenges facing patients affected by end-stage liver diseases. Patients with comorbidities and advanced age are either not considered candidates for OLT or are expected to have reduced post-transplant survival (3-5). Additionally, transplantation procedures are costly both financially and in terms of health care resources. For these reasons, cell-based transplantation has been proposed as a therapeutic alternative to OLT, or a bridge, as patients wait for an available organ (6).

To date, hepatocyte transplantation has demonstrated its functional utility in animal models. From the transgenic urokinase (7-9) to the induced tyrosinemic mouse (10-15), hepatocyte transplantation has successfully established its therapeutic potential with complete regeneration of the liver. In spite of these encouraging results, human hepatocyte transplantation is still in the experimental phase of clinical exploration (6, 16) in hope that the positive results of animal studies can be translated into therapeutic utility for human diseases.

However, for patients suffering from a terminal liver disease, there is an additional challenge: most of the envisioned cellular therapies are directed at cell engraftment in the diseased liver itself. Transplanted liver cells are generally injected via the spleen (e.g., through the splenic artery in human patients or into the splenic parenchyma in rodents) or via the portal vein. Liver cells rapidly migrate, actively or passively, to the diseased liver, where hepatic regeneration by the transplanted hepatocytes is expected to occur. This approach limits, and possibly precludes, the efficacy of cellular therapy in a vast majority of patients with severe liver disease due to the presence of cirrhosis and fibrosis (17), common pathological features in diseased livers. In consequence, native liver regeneration for these patients - the patients most in need of the treatment - is a major challenge.

Accordingly, there is a need for new therapies for patients with advanced liver disease. One option that has been explored is engineering an auxiliary liver (18-20). Generally, this consists of implanting a healthy liver graft placed either heterotopically or orthotopically while leaving all or part of the native liver intact. This approach not only has the potential to avoid OLT for a certain category of patients, but it also embraces the potential for spontaneous regeneration of the native liver and eventual withdrawal of immunosuppressive drugs (21-24). Although problems have been noted in early trials (18-20, 25-27), more recent favorable outcomes have been reported and are encouraging in cases of acute liver failure (25), metabolic disorder (28-31), and even cirrhotic liver (26, 27). To date, there is no understanding of the cellular and molecular mechanism necessary to keep the liver cells and auxiliary liver stable and viable long-term.

A number of researchers have transplanted hepatocytes at a variety of extrahepatic sites (37-40). Engraftment of hepatocytes at most extra-hepatic sites has been associated with variable results. It has previously been demonstrated that hepatocytes, transplanted in lymph nodes, generated functional auxiliary liver(s) able to restore liver functions long-term (over 6 months) in mice (see, for example, United States Patent No. 9,125,891). These results formed the basis for a new paradigm for tissue regeneration through the use of lymphatic sites as *in vivo* bioreactors to grow tissue or organ substitutes (32-36).

In addition to liver disease, kidney disease is prevalent in the population and results in over 47,000 deaths annually in the United States. Known therapeutic methods for treating kidney disease include rest, diet changes, pharmacotherapy, hemodialysis therapy and kidney transplantation and depend on the severity of the disease. Upon reaching renal failure, dialysis therapy such as hemodialysis or peritoneal dialysis or kidney transplantation is necessary. Although hemodialysis therapy allows the elimination of waste that accumulates in the body, the function of the damaged kidney does not recover. In the cases of renal failure, unless the patient undergoes a kidney transplant, the patient will have to continue dialysis therapy for their lifetime. However, there are many difficulties associated with kidney transplantation including a shortage of donors, difficulties in tissue compatibility and avoidance of rejection reaction. Accordingly, there is a need for new therapies for patients with reduced kidney function.

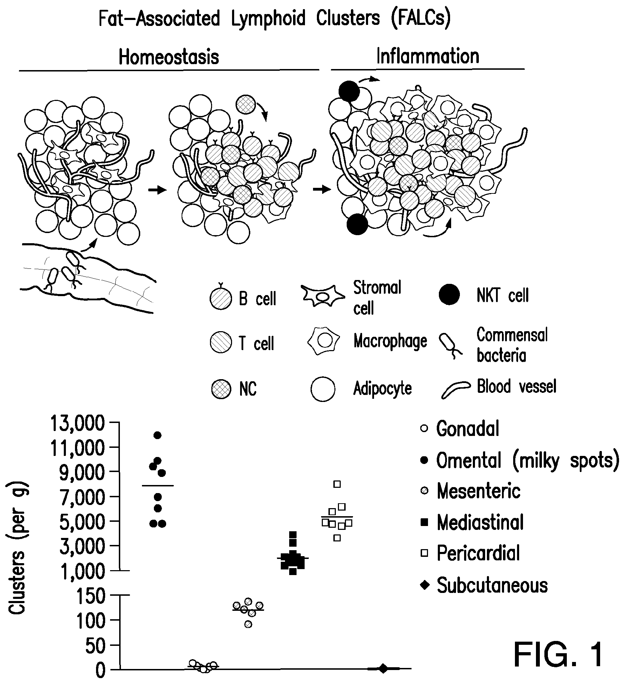

Lymph nodes and Fat-Associated Lymphoid Clusters ("FALCs," also referred to herein as "milky spots") are secondary lymphoid organs of the lymphatic system, and are well-vascularized. FALCs occur at a number of anatomical locations, including the omentum (see FIGURE 1). They have an important role in the immune system, allowing for massive expansion of white blood cells during various conditions such as bacterial or viral infections. Interestingly, lymph nodes and milky spots also have clinical significance in cancer, as they are the sites of early metastatic events, namely tumor invasion and initial metastatic growth. Tumor metastasis in lymph nodes is commonly used for the staging and prognosis of cancer.

US patent application publication number US2011002899A1 discloses methods of transplanting cells, such as hepatocytes, islet cells, and thymocytes at an ectopic location such as the lymph nodes, to treating liver disease as well as other disorders.

PCT application publication number WO2014138486A1discloses methods and compositions for transplanting non-lymphoid tissues (e.g. lung, kidney, thyroid, intestine, and brain) into lymphoid organs, which may be used to cultivate organ tissues including for the purpose of supplementing or reconstituting organ function.

US patent application publication number US2016058794A1 also discloses methods and compositions for transplanting non-lymphoid tissues (e.g. lung, kidney, thyroid, intestine, and brain) into lymphoid organs, which may be used to cultivate organ tissues including for the purpose of supplementing or reconstituting organ function.

The publication Bénézech et al., "Inflammation-induced formation of fat-associated lymphoid clusters", Nat Immunol 16, 819-828 (2015), doi:10.1038/ni.3215, discloses the association of FALCs with visceral fats.

### 3. SUMMARY OF THE INVENTION

The present disclosure relates to the engraftment and proliferation of cells in fat-associated lymphoid clusters ("FALCs" or "milky spots"), which may be used to generate functional ectopic tissue for transplantation into a host subject. It is based, at least in part, on the discovery that hepatocytes, intraperitoneally transplanted into a mouse with liver dysfunction, localize and proliferate in FALCs (milky spots) of the omentum, as well as in FALCs of mesenteric, splenic, portal and/or gonadal fat to produce ectopic liver tissue capable of beneficially augmenting liver function in the mouse. It is also based, in part, on the discovery that fetal kidney cells proliferate in FALCs of the omentum to produce ectopic kidney tissue. It is further based, in part, on the discovery that LTβR signaling in stromal cells promotes successful vascularization/angiogenesis of the grafted cells.

According to a first aspect of the present invention, there is provided a composition for use in generating an ectopic tissue in a fat-associated lymphoid cluster of a subject, according to claim 1 herein.

According to a second aspect of the present invention, there is provided a composition for use in generating an ectopic tissue in a fat-associated lymphoid cluster of a subject, according to claim 2 herein.

According to a third aspect of the present invention, there is provided a composition for use in generating an ectopic tissue in a fat-associated lymphoid cluster of a subject, according to claim 3 herein. The references to the methods of treatment by therapy in this description are to be interpreted as references to compounds, pharmaceutical compositions and medicaments of the present invention for use in those methods.

In certain embodiments, the present disclosure provides for methods and compositions to establish ectopic liver tissue in FALCs (milky spots) of the omentum, as well as in FALCs of mesenteric, splenic, portal and/or gonadal fat and to use such ectopic liver tissue for therapeutic benefit. In certain embodiments, the present disclosure further provides for methods and compositions to generate ectopic kidney tissue in FALCs, which can be used in a subject for therapeutic benefit.

In certain embodiments, the present disclosure further provides methods and compositions for grafting and proliferating cells, e.g., hepatocyte or kidney cells, in FALCs by activating the lymphotoxin beta receptor (LTβR) and/or NF-κB-inducing kinase (NIK) signaling pathway.

The present disclosure provides methods for developing ectopic tissue in a subject. In certain embodiments, the method includes introducing one or more cells into a fat-associated lymphoid cluster of the subject and providing one or more agents that promotes the proliferation and/or vascularization of the one or more cells to form an ectopic tissue. In certain embodiments, the one or more cells are hepatocytes and the ectopic tissue is ectopic liver tissue. In certain embodiments, the one or more cells are kidney cells and/or kidney tissue fragments and the ectopic tissue is ectopic kidney tissue. In certain embodiments, the fat-associated lymphoid cluster is located in the adipose tissue of the pleural and/or pericardial and/or peritoneal cavity. In the peritoneal cavity, the FALC can be located in the omentum and/or mesenteric and/or splenic and/or portal and/or gonadal fat of the subject.

In certain embodiments, the agent that promotes the formation of ectopic tissue comprises one or more of bone marrow-derived cells and/or stromal cells. In certain embodiment, the one or more agents comprise stromal cells, e.g., fibroblast cells. In certain embodiments, the stromal cells express one or more of podoplanin, NIK and/or LTβR. In certain embodiments, the stromal cells are treated with an activator of the LTβR and/or NIK signaling pathway.

In certain embodiments, the agent that promotes the formation of ectopic tissue comprises an activator of the LTβR and/or NIK signaling pathway, e.g., an activator of LTβR and/or NIK. In certain embodiments, the agent that promotes the formation of ectopic tissue comprises an activator of the non-canonical NF-κB signaling pathway. In certain embodiments, the activation of LTβR and/or NIK and/or the activation of the non-canonical NF-κB signaling pathway promotes the proliferation and/or vascularization of the one or more cells to form the ectopic tissue.

In certain embodiments, the agent that promotes the formation of ectopic tissue comprises an agent that promotes inflammation. In certain embodiments, inflammation is induced prior to introducing one or more cells in a fat-associated lymphoid cluster of the subject.

The present disclosure further provides methods of treating a subject in need of augmented liver function. In certain embodiments, the method comprises administering, to the subject, a therapeutically effective amount of hepatocytes, and promoting the proliferation of a hepatocyte in a fat-associated lymphoid cluster of the subject to form ectopic liver tissue. In certain embodiments, the formation of ectopic liver tissue in the fat-associated lymphoid cluster is promoted by administration of the hepatocytes locally to the anatomical region of the fat-associated lymphoid cluster. In certain embodiments, the fat-associated lymphoid cluster is located in the omentum and/or mesenteric and/or splenic and/or portal and/or gonadal fat.

In certain embodiments, the formation of ectopic liver tissue in the fat-associated lymphoid cluster is promoted by co-administration of one or more of bone marrow-derived cells or stromal cells. For example, but not by way of limitation, stromal cells are co-administered. In certain embodiments, the stromal cells are fibroblast cells. In certain embodiments, the stromal cells express one or more of podoplanin, NIK, and/or LTβR.

In certain embodiments, the formation of ectopic liver tissue in the fat-associated lymphoid cluster is promoted by co-administration of an activator of the LTβR and/or NIK signaling pathway. In certain embodiments, the formation of ectopic liver tissue in the fat-associated lymphoid cluster or lymph node is promoted by co-administration of an activator of the non-canonical NF-κB signaling pathway.

In certain embodiments, the formation of ectopic liver tissue in the fat-associated lymphoid cluster is promoted by inducing inflammation in the subject. In certain embodiments, inflammation is induced by administration of an agent that promotes inflammation to the subject. In certain embodiments, the inflammation is induced prior to introducing one or more cells in a fat-associated lymphoid cluster of the subject.

The present disclosure further provides methods for generating an ectopic liver, where the method comprises introducing, into a fat-associated lymphoid cluster one or more hepatocytes, and providing at least one agent that promotes the formation of ectopic liver tissue. In certain embodiments, the method is practiced *in vivo* or *in vitro.* In certain embodiment, the agent that promotes the formation of ectopic liver tissue comprises one or more of bone marrow-derived cells and/or stromal cells. In certain embodiments, the stromal cells are fibroblast cells. In certain embodiments, the stromal cells express one or more of podoplanin, NIK and/or LTβR. In certain embodiments, the stromal cells are treated with an activator of the LTβR and/or NIK signaling pathway. In certain embodiments, the agent that promotes the formation of ectopic tissue comprises an activator of the LTβR and/or NIK signaling pathway. In certain embodiments, the agent that promotes the formation of ectopic tissue comprises an agent that promotes inflammation.

In certain embodiments, the method for generating an ectopic liver tissue in a subject, comprises introducing cells comprising one or more hepatocytes and one or more stromal cells in a fat-associated lymphoid cluster of the subject and providing an activator of the LTβR and/or NIK signaling pathway, wherein activation of the LTβR and/or NIK signaling pathway in the one or more stromal cells promotes the proliferation and/or vascularization of the one or more hepatocytes to form the ectopic liver tissue.

In certain embodiments, the method for generating an ectopic liver tissue in a subject, comprises introducing cells comprising one or more hepatocytes and one or more stromal cells in a fat-associated lymphoid cluster of the subject and providing an activator of LTβR and/or NIK, wherein activation of LTβR and/or NIK in the one or more stromal cells promotes the proliferation and/or vascularization of the one or more hepatocytes to form the ectopic liver tissue. In certain embodiments, the kidney cells comprise cells isolated from embryonic kidney, metanephroi, cells isolated from a kidney organoid formed *in vitro* or any combination thereof.

The present disclosure further provides methods of treating a subject in need of augmented kidney function. In certain embodiments, the method comprises administering, to the subject, a therapeutically effective amount of kidney cells (or kidney tissue fragments), and promoting the proliferation of a kidney cell in a fat-associated lymphoid cluster of the subject to form ectopic kidney tissue. In certain embodiments, the formation of ectopic kidney tissue in the fat-associated lymphoid cluster is promoted by administration of the hepatocytes locally to the anatomical region of the fat-associated lymphoid cluster. In certain embodiments, the fat-associated lymphoid cluster is located in the omentum and/or mesenteric and/or splenic and/or portal and/or gonadal fat.

In certain embodiments, the formation of ectopic kidney tissue in the fat-associated lymphoid cluster is promoted by co-administration of one or more of bone marrow-derived cells or stromal cells. For example, but not by way of limitation, stromal cells are co-administered. In certain embodiments, the stromal cells are fibroblast cells. In certain embodiments, the stromal cells express one or more of podoplanin, NIK, and/or LTβR.

In certain embodiments, the formation of ectopic kidney tissue in the fat-associated lymphoid cluster is promoted by co-administration of an activator of the LTβR and/or NIK signaling pathway. In certain embodiments, the formation of ectopic kidney tissue in the fat-associated lymphoid cluster is promoted by co-administration of an activator of the non-canonical NF-κB signaling pathway.

In certain embodiments, the formation of ectopic kidney tissue in the fat-associated lymphoid cluster is promoted by inducing inflammation in the subject. In certain embodiments, inflammation is induced by administration of an agent that promotes inflammation to the subject. In certain embodiments, inflammation is induced prior to introducing the one or more cells kidney cells in a fat-associated lymphoid cluster of the subject.

The present disclosure further provides methods for generating an ectopic kidney, where the method comprises introducing, into a fat-associated lymphoid cluster one or more kidney cells (or one or more kidney tissue fragments), and providing at least one agent that promotes the formation of ectopic kidney tissue. In certain embodiments, the method is practiced *in vivo* or *in vitro.* In certain embodiments, the agent that promotes the formation of ectopic kidney tissue comprises one or more of bone marrow-derived cells and/or stromal cells. In certain embodiments, the stromal cells are fibroblast cells. In certain embodiments, the stromal cells express one or more of podoplanin, NIK and/or LTβR. In certain embodiments, the stromal cells are treated with an activator of the LTβR and/or NIK signaling pathway. In certain embodiments, the agent that promotes the formation of ectopic kidney tissue comprises an activator of the LTβR and/or NIK signaling pathway. In certain embodiments, the agent that promotes the formation of ectopic kidney tissue comprises an agent that promotes inflammation.

In certain embodiments, a method for generating an ectopic kidney tissue in a subject, comprises introducing cells comprising one or more kidney cells and one or more stromal cells in a fat-associated lymphoid cluster of the subject and providing an activator of the LTβR and/or NIK signaling pathway, wherein activation of the LTβR and/or NIK signaling pathway in the one or more stromal cells promotes the proliferation and/or vascularization of the one or more kidney cells to form the ectopic kidney tissue.

In certain embodiments, a method for generating an ectopic kidney tissue in a subject comprises introducing cells comprising one or more kidney cells and one or more stromal cells in a fat-associated lymphoid cluster of the subject and providing an activator of LTβR and/or NIK, wherein activation of LTβR and/or NIK in the one or more stromal cells promotes the proliferation and/or vascularization of the one or more kidney cells to form the ectopic kidney tissue.

The present disclosure further provides compositions for generating an ectopic tissue. In certain embodiments, the composition comprises a plurality of cells and one or more of the agents that promote the formation of an ectopic tissue. In certain embodiments, a composition for generating an ectopic liver tissue comprises a plurality of hepatocytes and one or more of the agents that promote the formation of an ectopic liver. In certain embodiments, a composition for generating an ectopic kidney tissue comprises a plurality of kidney cells and one or more of the agents that promote the formation of an ectopic kidney.

In certain embodiments, the one or more agents that is included in a composition of the presently disclosed subject matter comprises a plurality of bone marrow-derived cells and/or a plurality of stromal cells. In certain embodiments, the stromal cells express one or more of podoplanin, NIK, and/or LTβR. In certain embodiments, the one or more agents is an activator of the LTβR and/or NIK signaling pathway. In certain embodiments, the one or more agents is an agent that promotes inflammation. In certain embodiments, the one or more agents comprises two or more of: (a) plurality of bone marrow-derived cells and/or a plurality of stromal cells stromal cells; (b) an activator of the LTβR and/or NIK signaling pathway; and (c) an agent that promotes inflammation. In certain embodiments, the composition further comprising a synthetic culture medium.

The invention is set out in the appended set of claims.

The present disclosure further provides kits comprising one or more compositions disclosed herein.

### 4. BRIEF DESCRIPTION OF THE FIGURES

FIGURE 1. Fat-associated lymphoid clusters; schematic depiction (upper panel), and distribution (lower panel).

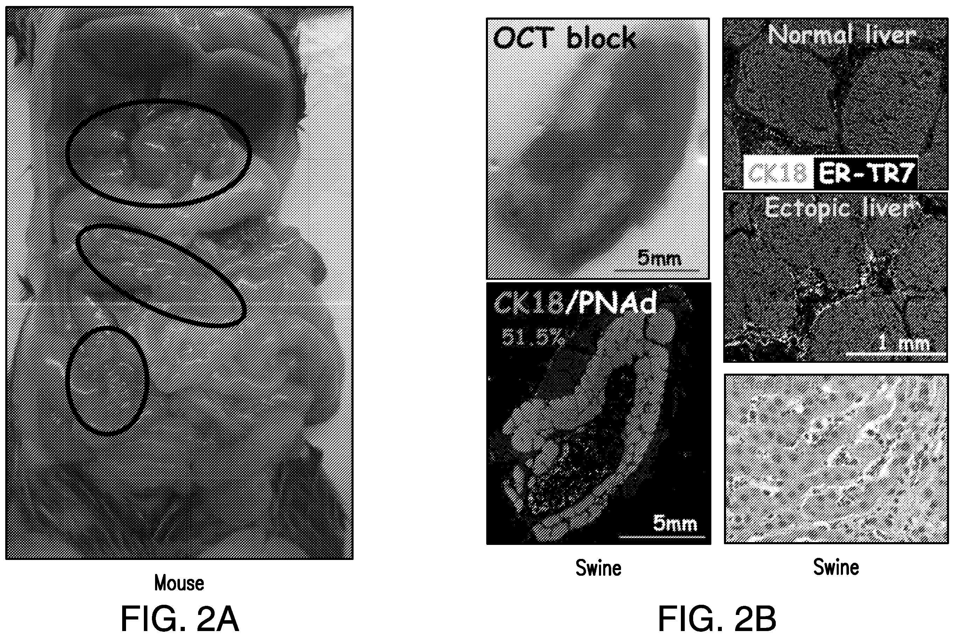

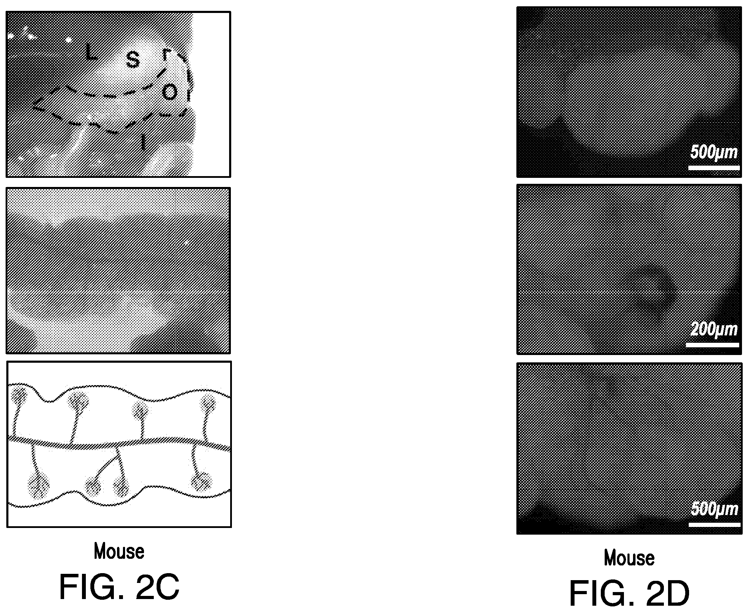

FIGURE 2A-D: Generation of ectopic livers in secondary lymphoid tissues. (A) Macroscopic image of a Fah^{-/-} mouse 12 weeks after IP transplantation of hepatocytes. Yellow circles highlight the many ectopic hepatic nodules generated in lymphoid tissues (milky spots and lymph nodes (LN)). (B) Mesenteric pig lymph node 2 months after portacaval shunt, partial hepatectomy and hepatocyte transplantation. Left panels, OCT block and frozen section from this block stained with CK18 for hepatocytes and PNAd for High Endothelial Venule in LN. 51.5% of the LN mass was identified as CK 18+ hepatocytes. Right panels, frozen sections of control liver and ectopic liver stained with CK18 (hepatocytes) and ER-TR7 (Fibroblast). H&E shows normal microvascularization present in the pig ectopic liver (C) Top panel, macroscopic view of mouse peritoneal space showing the greater omentum (O) covering the stomach (S) and intestine (I), adjacent to the liver (L) and above the pancreas (not shown). Middle panel, mouse omentum under dissecting microscope showing abundant vasculature. Lower panel, cartoon depiction of the omentum showing vascular trees feeding the milky spots. (D) IP transplantation of GFP+ hepatocyte. Ectopic nodules were generated in Fah-/- mice milky spots 12 weeks after hepatocytes engrafted.
FIGURE 3. Fah^{-/-} mice 3 to 4 weeks after IP transplantation of hepatocytes; dilation of capillary space and sprouting of blood vessels in milky spots.
FIGURE 4. Omental milky spots in a mouse.
FIGURE 5. Engraftment of transplanted hepatocytes in omental milky spots. The hepatocytes are GFP labeled and present as bright areas of green fluorescence. ER-TR7 is an antigen found in the extracellular matrix of lymphoid tissues, fluorescent blue in this image (indicated by arrows).
FIGURE 6. Omental milky spots are lacking in FRGN (Fah^{-/-}Rag2/γc^{-/-}Nod) mice. Immunofluorescent staining of wild type (left) and FRGN omentum, milky spots ("MS") labeled.
FIGURE 7. Engraftment of hepatocytes in omentum of FRGN mice.
FIGURE 8. Engrafted hepatocytes in the omentum are not observed to grow in FRGN mice over a 12 week period.
FIGURE 9. Milky spots are restored in the omentum of FRGN mice by bone marrow transplantation.
FIGURE 10. Milky spots are restored in the omentum of FRGN mice by bone marrow transplantation. Note arrow in right panel showing engrafted hepatocytes in milky spot.
FIGURE 11. Ectopic liver formation in FRGN mice after bone marrow transplantation.
FIGURE 12. Ectopic liver formation in FRGN mice after bone marrow transplantation. Left panel shows result without bone marrow transplantation - hepatocyte aggregation but no ectopic liver formation is seen. Right panel shows result with bone marrow transplantation, generation of an ectopic liver.
FIGURE 13. Stromal cells/Lymphoid tissue inducer interaction (left) and NIK signal pathway (right).
FIGURE 14. Schematic of studies to test the impact of NIK function on rescue of liver function by intraperitoneal (IP) versus splenic (SP) injection in NIK-deficient (aly/aly) and control Fah^{-/-} mice. Kaplan Meier survival curves of Fah-/- mice and aly/aly Fah-/- mice transplanted with 10⁶ hepatocytes by splenic (SP) injection (left) or IP injection (right).
FIGURE 15. NIK function is required to rescue the Fah^{-/-} mouse by IP injection. 10⁶ GFP+ hepatocytes were inoculated by IP injection into Fah^{-/-} mice and into the alymphoplasia Fah^{-/-} mice (aly/aly Fah^{-/-}) respectively. Whole-mount imaging of omental milky spots at various time points. Shown are the bright-field images merged with the fluorescence of donor hepatocytes (green). Top panels, Fah^{-/-} mouse; bottom panels, aly/aly Fah^{-/-} mouse.
FIGURE 16. Schematic representation of the observed outcome after IP hepatocyte transplantation.
FIGURE 17A-D. (A) Distribution of podoplanin (PDPLN) and CD31 in fibroblastic reticular cells ("FRC"), lymphatic endothelial cells (LEC), and brain endothelial cells (BEC), and flow cytometric analysis of these markers in lymph nodes as compared to milky spots. (B) Hepatocytes establish themselves in close proximity to ER-TR7 stromal cells in milky spots. (C) Isolated stromal cells, transplanted IP, will migrate back to omental milky spots. (D) Milky spot with transplanted hepatocytes.
FIGURE 18. Immunofluorescent studies demonstrating that hepatocytes are lymphotoxin alpha and beta positive and stromal cells are NIK and lymphotoxin beta receptor positive.
FIGURE 19. Wild-type GFP+ (Green Fluorescent Protein) hepatocytes transplanted intraperitoneally into either Fah^{-/-} or Fah/LTbR^{-/-} mice after 2, 4 and 6 weeks. The pictures show engraftment in the omentum of these animals.

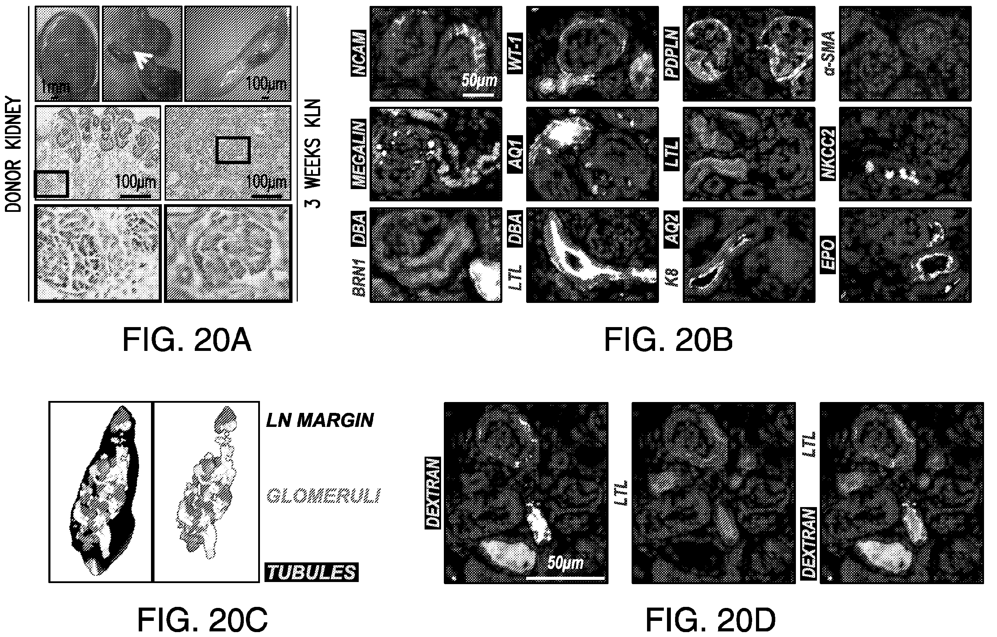

FIGURE 20A-G. (A) Top, from left to right, images of human fetal kidney, jejunal LNs after kidney fragment transplantation, and LNs 3 weeks after transplantation. Middle left, H&E-stained section of paraffin-embedded donor human fetal kidney. Middle right, hematoxylin-stained frozen section of LN 3 weeks after kidney transplantation. The region enclosed by the orange dashed line indicates non-engrafted LN's area. Bottom, the insets show a non-mature (left) or a mature (right) glomerulus. (B) Representative immunofluorescence stainings of 3-week graft sections for NCAM, WT-1, PDPLN, α-SMA, Megalin, AQ1, LTL, NKCC2, BRN1/DBA, LTL/DBA, K8/AQ2 or EPO. (C) Representative 3D reconstruction of an 8-week graft. Left, image of engrafted glomeruli and tubules inside the LN. Right, image with glomeruli and tubules only. (D) 10,000 kDa MW Texas Red Dextran accumulation in 11-week mouse bearing graft. (E) Representative immunofluorescence stainings of 1-, 3- and 8-week graft sections for mouse and human CD31. (F) Left, representative immunofluorescence stainings for NCAM, SIX2, WT-1 or E-CADH in a mouse NP organoid before transplantation. Right, representative immunofluorescence stainings for PDPLN/CD31, PDPLN/CD105, or PDPLN/Ly-76 showing vascularized glomerulus-like structures in LN 8 weeks after NP organoid transplantation. (G) Left, representative whole-mount immunofluorescence stainings of iPSC-derived kidney organoids for DBA/BRN1 and E-CADH/LTL. Bottom, serial sections showing two distinct nephron-like structures, each comprised by a glomerular-like structure containing presumptive podocytes (PDPLN, SYNPO, PODXL), mesangial cells (CIV), and endothelial cells (CD31), adjacent to LTL/Megalin reactive proximal-like tubules, 6 week after organoid transplantation into the LN. Nuclei were counterstained using Hoechst (blue).
FIGURE 21. Schematic representation of the canonical and non-canonical NF-κB signaling pathways.

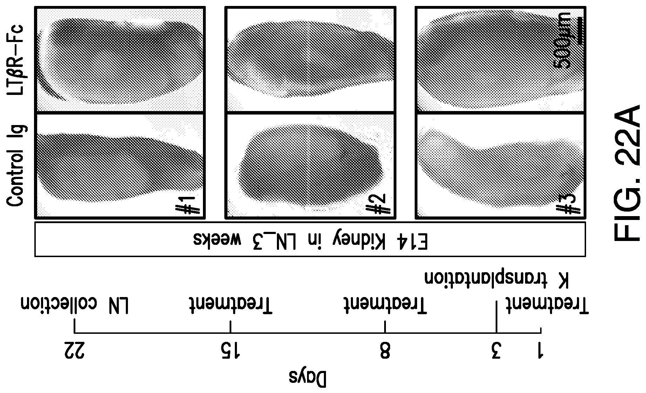

FIGURE 22A-G. (A) Left, schematic view of the experimental plan. Mice were injected IP with 100µg LTβR-Fc or Control Ig two days before receiving transplantation of embryonic GFP+ kidney fragments into their LNs (Day 1). Mice were treated again at Day 8 and 15. LNs were collected at Day 22. Right, whole mount kidney-bearing LNs isolated from treated or untreated mice. (B) Representative immunofluorescence stainings of serial sections of kidney grafts as in A for PDPLN, LTL, CD31, CD105, Ly-76. (C) Flow cytometric profiles of fibroblast reticular cells (FRCs), lymphatic endothelial cells (LECs), and blood endothelial cells (BECs) in LN or omental stromal cell population (CD45-). (D) Representative immunofluorescence staining for PDPLN and LTβR of omental stromal cells. (E) Whole mount kidney-bearing omenta from wild type or LTβR-/- mice after transplantation of embryonic GFP+ kidneys, 6 weeks after transplantation (merged fluorescence/bright-field images on the left and dissecting scope images on the right). Neo-kidneys are shown in comparison to an adult mouse kidney. (F) Representative immunofluorescence stainings for CD31, CD105, and Ly-76 on serial sections of intraomental kidney grafts as in E. (G) Representative immunofluorescence stainings for ERTR-7, LTα, LTβ, LTβR, NIK, NIK/CD34 on sections of embryonic GFP+ (left and middle) or wild type (right) kidney LN grafts. Nuclei were counterstained using Hoechst (blue).
FIGURE 23. Peritoneal inflammation induces dramatic changes in the omentum. Upper panel. Schematic view of the experiment. Zymosan is injected intraperitoneally (IP) on day 0. Three days later, 1 million hepatocytes are injected IP. 7 days after hepatocyte injection the samples are collected. Lower left panel. Macroscopic view of the omentum from a zymosan induced and control animal. Lower right panel. Dramatic increase in the weight of the omentum after Zymosan induced inflammation when compared to control.
FIGURE 24. FALCs and hepatic growth. Omentum and mesenteric fat of C57bl/6 mice after zymosan or PBS injection one week after GFP+ hepatocyte transplantation. Zymosan inflammation induced FALCs number and size that allowed a dramatic increase in GFP+ hepatocyte engraftment. Omentum and mesenteric fat were compared and GFP+ hepatocytes identified under fluorescent microscope.

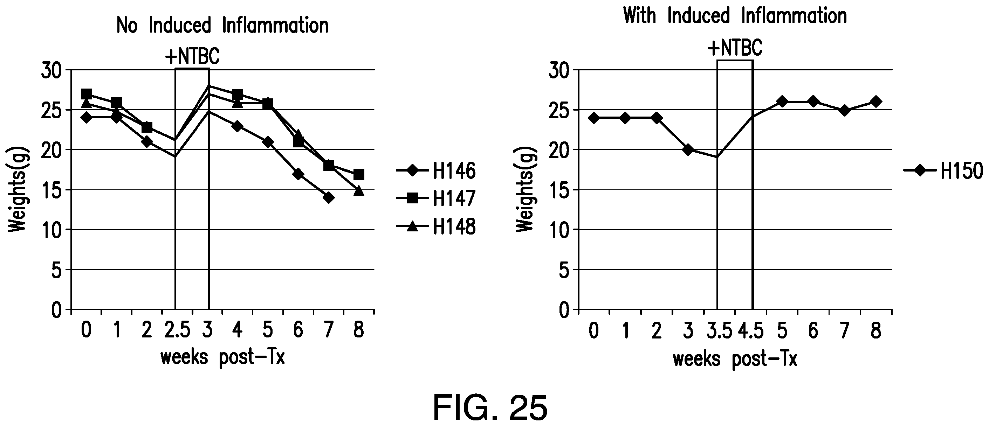

FIGURE 25. Peritoneal inflammation increases survival of tyrosinemic mice after hepatocyte transplantation. Fah^{-/-} C57bl/6 mice with or without previous induced inflammation were transplanted IP with wild type hepatocytes followed by two selections (off NTBC). Animals without inflammation (left panel) continued to lose weight after the second selection, Animal H146 and H148 died around/at 8 weeks after transplantation. The animal with previous induced inflammation (right panel) increased weight during the second selection indicating a rescue of liver disease with restoration of functional liver mass.
FIGURE 26. Peritoneal inflammation increases liver mass present in fat containing FALCs. A macroscopic view of the peritoneal fat containing liver tissues from Fah^{-/-} C57bl/6 mice without (H146) and with (H150) previous induced inflammation. Inflammation is supportive of ectopic liver growth in FALCs.

### 5. DETAILED DESCRIPTION OF THE INVENTION

For clarity and not by way of limitation, the detailed description of the presently disclosed subject matter is divided into the following subsections:
(i) Definitions;
(ii) Treatment;
   a. Treatment of Liver Diseases and Disorders;
   b. Treatment of Kidney Diseases and Disorders; and
(iii) Compositions.

### 5.1 DEFINITIONS

Unless otherwise defined, all technical and scientific terms used in this application have the same meaning as commonly understood by one of ordinary skill in the art.

As used herein, the term "about" or "approximately" means within an acceptable error range for the particular value as determined by one of ordinary skill in the art, which will depend in part on how the value is measured or determined, *i.e.,* the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

Fat-Associated Lymphoid Clusters ("FALCs," alternatively referred to as "milky spots" herein), as used herein, are structures of the lymphoid system that are not lymph nodes. FALCs are present in a number of anatomical locations, including but not limited to the mesentery, the mediastinum, the pericardium and subcutaneous tissue (see FIGURE 1). FALCs are located in the adipose tissue of the pleural, pericardial and peritoneal cavity. In the peritoneal cavity, the FALC is located in the omentum and mesenteric, splenic, portal and gonadal fat of the subject. In certain non-limiting embodiments, the FALCs used for ectopic tissue growth, e.g., liver or kidney tissue growth, is located in the omentum. In certain non-limiting embodiments, the FALCs are located in the mediastinum or pericardium.

"Lymph Node" or "LN," as used herein, refers to any lymph node for example, but not limited to, abdominal lymph nodes, celiac lymph nodes, paraaortic lymph nodes, splenic hilar lymph nodes, porta hepatis lymph nodes, gastric lymph nodes (left and right), gastroomental (gastroepiploic) lymph nodes (left and right), retroperitoneal lymph nodes, pyloric lymph nodes (suprapyloric, subpyloric, retropyloric), pancreatic lymph nodes (superior pancreatic, inferior pancreatic, splenic lineal lymph nodes), hepatic lymph nodes (cystic, foraminal-including foramen of Winslow), pancreaticoduodenal lymph nodes (superior pancreaticoduodenal, inferior pancreaticodoudenal), superior mesenteric lymph nodes, ileocolic lymph nodes, prececal lymph nodes, retrocecal lymph nodes, appendicular lymph nodes, mesocolic lymph nodes (paracolic, left colic, middle colic, right colic, inferior mesenteric lymph nodes, sigmoid, superior rectal), common iliac lymph nodes (medial common ilic, intermediate common iliac, lateral common iliac, subaortic common iliac, common iliac nodes of promontory), and external iliac lymph nodes (medial external iliac, intermediate external iliac, lateral external iliac, medial lacunar-femoral, intermediate lacunar-femoral, lateral lacunar-femoral, interiliac external iliac, obturator-external iliac obturatory), Jejunal, popliteal and axillary lymph nodes.

Cells of an organ may be comprised of one or a plurality of cell types. In certain embodiments, cells are comprised of a plurality of cell types found in the originating organ, but need not necessarily comprise all cell types found in that organ. As one non-limiting example, "liver cells" for engraftment according to the present disclosure comprise hepatocytes and may further comprise one or more of biliary cells, endothelial cells, stem cells and progenitor cells of the liver. As another non-limiting example, "kidney cells" for engraftment according to the present disclosure comprise renal parenchymal cells and may further comprise one or more of glomerular cells, endothelial cells, stem and progenitor cells of the kidney. In certain embodiments, the cells are dissociated prior to introduction for engraftment. In certain embodiments, the cells are comprised in an aggregate prior to introduction. In certain embodiments, the cells are comprised of organoids expanded *in vitro.* In certain embodiments, the cells are comprised of iPS or other stem cells expanded *in vitro.* In certain embodiments, the cells are comprised in a tissue fragment obtained from an intact organ, but said fragment constitutes no more than about 5%, or no more than about 10%, or no more than about 25% or no more than about 50% of the organ.

"Activator" as used herein, refers to a compound or molecule (*e.g*., small molecule, peptide, peptidomimetic, natural compound or antibody) that activates (*e.g.*, increases, promotes or enhances) the activity, function, expression and/or generation of a protein or pathway. An activator can be any compound or molecule that promotes any activity of a named protein (molecule, any molecule involved with the named molecule or a named associated molecule), such as LTβR or NIK, or promotes the interaction of a named protein, *e.g.,* LTβR or NIK, with signaling partners or binding partners. Activators can also include molecules that indirectly regulate the biological activity of a named protein, *e.g.,* LTβR or NIK, by intercepting upstream signaling molecules. In certain embodiments, the activator can include molecules that promote, increase and/or enhance the generation and/or production of a named protein such as LTβR or NIK, *e.g.,* by increasing the activity and/or function of the enzymes that generate and/or produce LTβR or NIK.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishing any direct or indirect pathological consequences of the disease, decreasing the rate of disease progression, amelioration or palliation of the disease state and remission or improved prognosis. In certain embodiments, "treatment" can refer to a decrease in the severity of complications, symptoms and/or deterioration. For example, but not by way of limitation, the decrease can be a 10%, 20%, 30%, 40%, 50%, 60%, 70%, 80%, 90%, 95%, 98% or 99% decrease in severity of complications, symptoms and/or deterioration, for example relative to a comparable control subject not receiving the treatment. In certain embodiments, "treatment" can also mean prolonging survival as compared to expected survival if treatment is not received.

A "therapeutically effective amount" or "effective amount" as used herein, refers to an amount that is able to achieve one or more of the following: alleviation of symptoms, decreasing the rate of disease progression, prolongation of survival, amelioration or palliation of the disease state and/or improved prognosis.

A "subject" or "individual," as used interchangeably herein, can refer to a human or a non-human subject. Non-limiting examples of non-human subjects include non-human primates, dogs, cats, horses, mice, rats, hamsters, rabbits, swine, etc.

The term or phrase "transplantation," "cell replacement" or "grafting," as used interchangeably herein, refer to the introduction of cells to a target tissue, *e.g*., FALCs or tissues that contain FALCs.

"In combination with," as used herein, can mean that one or more cells to be grafted or a composition thereof, and an agent, *e.g.,* an activator of the LTβR and/or NIK signaling pathway, are administered to a subject as part of a treatment regimen or plan. In certain embodiments, being used in combination does not require that the one or more cells and the one or more agents are physically combined prior to administration or that they be administered over the same time frame. For example, but not by way of limitation, the one or more cells to be grafted and the one or more agents can be administered concurrently to the subject being treated, or can be administered at the same time or sequentially in any order or at different points in time. In certain embodiments, methods of the present disclosure can include the administration of one or more agents prior to administration of the one or more cells to be grafted, *e.g*., hepatocytes or kidney cells.

### 5.2TREATMENT

In certain embodiments, the present disclosure relates to the engraftment and proliferation of cells in fat-associated lymphoid clusters ("FALCs" or "milky spots") to develop ectopic tissue.

The present disclosure provides methods of generating ectopic tissue in or around a FALC. In certain embodiments, the method for generating ectopic tissue can comprise introducing a cell, *e.g.,* a hepatocyte or a kidney cell, into a FALC of a subject to form an ectopic tissue, *e.g.,* an ectopic liver tissue or an ectopic kidney tissue. In certain embodiments, the methods for generating an ectopic tissue can comprise introducing a cell in a lymph node of the subject. In certain embodiments, the methods of the present disclosure can be used to treat a medical condition and/or disorder (*e.g.,* pathology, disease, syndrome) which may benefit from the generation of the ectopic tissue. For example, but not by way of limitation, the subject can be suffering from a disease or disorder such as, but not limited to, liver disease and/or failure or kidney disease and/or failure.

In certain embodiments, the subject can be human or non-human. Non-limiting examples of non-humans include non-human primates, dogs, cats, horses, mice, rats, hamsters, rabbits, swine, etc. In certain embodiments, the subject is human.

In certain embodiments, the cells to be grafted into the FALCs of a subject can be human cells, non-human cells or both. In certain embodiments, the cells are human cells. In certain embodiments, human cells can be grafted into the FALCs, or a region that contains FALCs, of a non-human subject, *e.g*., mouse.

The cells for engraftment can be obtained from the subject that is undergoing the graft. In certain embodiments, the cells can be obtained from a source other than the subject that is undergoing the graft. In certain embodiments, the cells can be obtained from fresh or frozen cell populations. In certain embodiments, the cells can be isolated from tissue and grown *in vitro* under various culture conditions prior to transplantation. In certain embodiments, the cells can be obtained from embryonic, fetal, pediatric or adult tissue. In certain embodiments, the cells can be progenitor or precursor cells. In certain embodiments, the progenitor or precursor cells can be differentiated into the type of cell to be transplanted.

The cells for use according to the present disclosure can be prepared by any means known in the art. In certain embodiments, cell solutions, *e.g.*, compositions disclosed herein, are used for injection. Non-limiting examples of compositions for use in the disclosed methods are described in Section 5.3 below. In certain embodiments, the cells can be prepared according to the method of Li et al. J Tissue Culture Methods 14:139-146 (1992). Briefly, isolation involves collagenase perfusion of a sample of limited size (<50 g) with only one cut surface. In certain embodiments, specifically enriched cell populations can be used such as those disclosed in U.S. Pat. No. 7,211,404.

The cells can be administered to the subject by any method known in the art. In certain embodiments, the cells can be injected, including but not limited to, intraperitoneal, intravenous or intraarterial injection or by local instillation. In certain embodiments, the cells can be surgically engrafted, *e.g*., by conventional or endoscopic surgical techniques. In certain embodiments, the cells can be locally administered. For example, but not by way of limitation, cells can be introduced into the proximity of an anatomical location that contains FALCs, such as, but not limited to, the gonads, the omentum, the mesentery, the mediastinum, the pericardium and subcutaneous tissue. In certain embodiments, the anatomical region is the omentum. In certain embodiments, *e.g*., hepatocytes or kidney cells (or fragments of kidney tissue), can be introduced into proximity of the omentum (and FALCs residing there), for example by targeted intraperitoneal injection or by conventional or endoscopic surgical techniques. In certain embodiments, the cells to be engrafted may be initially expanded in culture before introduction into a FALC or lymph node *in vivo.*

In certain embodiments, the cells can be suspended in any appropriate buffer for injection. Non-limiting examples of such buffers include saline, phosphate buffered saline, Hank's salt and Ringer's solution, among others.

In certain embodiments, the method for generating an ectopic tissue in or around a FALC can further comprise providing an agent that promotes the formation of the ectopic tissue. For example, but not by way of limitation, the methods can include administering one or more cells to be grafted in combination with one or more agents that promote the formation of the ectopic tissue. In certain embodiments, the one or more agents can be included in the composition that includes the one or more cells to be grafted. Alternatively and/or additionally, the one or more agents can be administered prior to, after or concurrently with the grafting of the one or more cells. In certain embodiments, the agent promotes the proliferation and/or vascularization of the one or more cells to form the ectopic tissue. Analogous methods may be used to generate an ectopic tissue in a lymph node.

In certain embodiments, the agent that promotes the formation of ectopic tissue can be a cell type different from that of the cells, *e.g.,* kidney cells or hepatocytes, that form the ectopic tissue. In certain embodiments, the agent includes one or more of bone marrow-derived cells and/or stromal cells. In certain embodiments, the agent includes stromal cells, *e.g*., fibroblasts, fibroblastic reticular cells (FRCs), folicular dendritic cells (FDCs), lymphatic endothelial cells (LECs), blood endothelial cells (BECs), alpha-7 integrin pericytes (AIPs) and double negative cells (DNCs). In certain embodiments, the fibroblasts can be human foreskin fibroblasts, human embryonic fibroblasts, mouse embryonic fibroblasts, skin fibroblasts cells, vascular fibroblast cells, myofibroblasts, smooth muscle cells, mesenchymal stem cells (MSCs)-derived fibroblast cells or a combination thereof. For example, but not by way of limitation, a method for generating ectopic tissue can comprise (a) introducing one or more cells, *e.g.,* hepatocytes or kidney cells, into a FALC (or an anatomical region containing the FALC) of a subject to form an ectopic tissue, *e.g.,* an ectopic liver tissue or an ectopic kidney tissue, and (b) introducing one or more bone marrow-derived cells and/or stromal cells to promote the formation of ectopic tissue, *e.g.,* by promoting the proliferation and/or vascularization of the one or more cells to form the ectopic tissue. In certain embodiments, the one or more cells to be grafted and the one or more bone marrow-derived cells and/or stromal cells can be included in the same composition.

In certain non-limiting embodiments, the stromal cells, *e.g.,* stromal endothelial cells or fibroblasts, that promote the formation of ectopic tissue may express detectable levels of one or more of podoplanin, lymphotoxin beta receptor ("LTβR"), NIK or a combination thereof. Alternatively and/or additionally, the stromal cells can be treated with an activator of the LTβR and/or NIK signaling pathway, *e.g.,* an activator of LTβR or NIK or downstream targets thereof, prior to, during or after administration to the subject. In certain embodiments, stromal cells, *e.g.,* stromal endothelial cells or fibroblasts, can be treated with an activator of NIK. In certain embodiments, the stromal cells can be genetically modified to exogenously express LTβR and/or NIK and/or overexpress LTβR and/or NIK.

In certain embodiments, the agent that promotes the formation of ectopic tissue, *e.g.,* by promoting the proliferation and/or vascularization of the one or more cells to form the ectopic tissue, can be an activator of the LTβR and/or NIK signaling pathway. For example, but not by way of limitation, the method of generating ectopic tissue in or around a FALC can comprise introducing a cell, *e.g.,* a hepatocyte or a kidney cell into a FALC (or an anatomical region containing the FALC) of a subject and providing at least one activator of the LTβR and/or NIK signaling pathway, wherein the activation of LTβR and/or NIK or downstream targets thereof promotes formation of the ectopic tissue, *e.g.,* an ectopic liver tissue or an ectopic kidney tissue. In certain embodiments, the activator of the LTβR and/or NIK signaling pathway is an activator of LTβR and/or NIK or an activator of the non-canonical NF-κB signaling pathway. In certain embodiments, the one or more cells to be grafted and the activator of the LTβR and/or NIK signaling pathway can be included in the same composition. Alternatively, the activator can be administrated prior to, during or after introduction of the one or more cells to be grafted into the subject.

In certain embodiments, the activator of the LTβR and/or NIK signaling pathway can increase expression of LTβR or NIK in a cell, *e.g.,* a stromal cell or stromal endothelial cell, present in the subject that is undergoing the graft. For example, but not by way of limitation, the activator of the LTβR and/or NIK signaling pathway activates the signaling pathway in stromal cells present in the FALC of the subject.

Alternatively and/or additionally, the cells to be grafted can be a heterogeneous cell population, *e.g.,* a composition comprising a heterogeneous cell population, that includes stromal cells or stromal endothelial cells, and the activator of the LTβR and/or NIK signaling pathway can increase expression of LTβR or NIK in the stromal cells or stromal endothelial cells present in the composition to be grafted. For example, but not by way of limitation, a method for generating ectopic tissue can comprise (a) introducing one or more cells, *e.g.,* hepatocytes or kidney cells, into a FALC of a subject to form an ectopic tissue, *e.g.,* an ectopic liver tissue or an ectopic kidney tissue, (b) introducing one or more bone marrow-derived cells and/or stromal cells to promote the proliferation and/or vascularization of the one or more cells to form the ectopic tissue and (c) administering an activator of the LTβR and/or NIK signaling pathway. In certain embodiments, the activator of the LTβR and/or NIK signaling pathway is present in the composition that includes the one or more cells to be grafted and the one or more bone marrow-derived cells and/or stromal cells or the activator of the LTβR and/or NIK signaling pathway is present in the composition that includes the one or more bone marrow-derived cells and/or stromal cells.

In certain embodiments, the activator of the LTβR and/or NIK signaling pathway can be an activator of LTβR or a downstream target of LTβR. In certain embodiments, the activator can be an activator of NIK or a downstream target of NIK, *e.g.,* an activator of the non-canonical NF-κB signaling pathway. In certain embodiments, the activator of the LTβR and/or NIK signaling pathway can be an agonist antibody (or antibody fragment thereof) or single chain antibody that specifically binds to LTβR or NIK. Non-limiting examples of anti-LTβR agonist antibodies include the monoclonal antibody produced by Adipogen Life Sciences., catalog number AG-20B-0008, the anti-LTβR agonist antibody CBE11 (see, *e.g.,* Lukashev et al., Cancer Res. 66(19):9617-9624 (2006)), the anti-LTβR agonist antibody BS-1 (see, *e.g.,* Hu et al., Carcinogenesis 34(5):1105-1114 (2013)) and the anti-LTβR agonist antibody 4H8 (see, *e.g.,* Scarzello et al., Gut 65:1765-1775 (2016)). Additional non-limiting examples of anti-LTβR agonist antibodies are disclosed in U.S. Patent Publication No. 2002/0090366, the contents of which are disclosed herein in its entirety. In certain embodiments, the activator of the LTβR and/or NIK signaling pathway can be a small molecule that increases function and/or activity of LTβR or NIK or a downstream target thereof

In certain embodiments, the present disclosure provides a method for generating an ectopic tissue in a subject comprising introducing one or more cells into a FALC or lymph node of the subject and providing an activator of LTβR and/or NIK, wherein activation of LTβR and/or NIK promotes the proliferation and/or vascularization of the one or more cells to generate the ectopic tissue. In certain embodiments, the one or more cells comprise a hepatocyte and the ectopic tissue comprises an ectopic liver tissue. In certain embodiments, the one or more cells comprise a kidney cell and the ectopic tissue comprises an ectopic kidney tissue. In certain embodiments, the one or more cells further comprise one or more stromal cells and the activator of LTβR and/or NIK results in activation of the LTβR and/or NIK signaling pathway in the stromal cells.

In certain embodiments, the agent that promotes the formation of ectopic tissue can be an agent that promotes inflammation in the subject. For example, but not by way of limitation, the method for generating an ectopic tissue in or around a FALC can further include inducing inflammation in the subject, e.g., by administration of an agent that promotes inflammation in the subject. Non-limiting examples of such agents include cytokines, vasodilators, histamine, serotonin, bradykinin and zymosan. Non-limiting examples of cytokines include IL-1, IL-2, IL-3, IL-4, IL-5, IL-6, IL-7, IL-8, IL-9, IL-10, IL-11, IL-12, IL-13, IL-15, IL-17, IL-18, IL-21, IL-23, IL-24, IL-27, IFN-alpha, IFN-alpha2, IFN-beta, or IFN-gamma, TNF-alpha and TNF-beta, TGF-alpha and TGF-beta, Lymphotoxin-alpha and Lymphotoxin-beta. Non-limiting examples of chemokines include CCL18, CCL19, CCL21, CXCL8, CCL2, CCL3, CCL4, CCL5 CCL7, CXCL12 and CXCL13. In certain embodiments, the agent comprises a compound and/or molecule that activates the complement system. In certain embodiments, the agent comprises zymosan. In certain embodiments, the agent is administered in and/or around the FALCs that were or are to be grafted with the cells. In certain embodiments, inflammation is induced locally, *e.g.,* in or around the anatomical region that contains FALCs where the cells were or are to be transplanted. Alternatively and/or additionally, the inflammation is induced systemically.

In certain embodiments, the inflammation is temporary. For example, but not by way of limitation, the inflammation lasts for no longer than about 10 weeks, no longer than about 9 weeks, no longer than about 8 weeks, no longer than about 7 weeks, no longer than about 6 weeks, no longer than about 5 weeks, no longer than about 4 weeks, no longer than about 3 weeks, no longer than about 2 weeks, no longer than about 1 week, no longer than about 6 days, no longer than about 5 days, no longer than about 4 days or no longer than about 3 days.

In certain embodiments, inflammation is induced prior to the introduction of the one or more cells to be grafted. For example, but not by way of limitation, inflammation is induced at least 2 hours before, at least 4 hours before, at least 6 hours before, at least 8 hours before, at least 10 hours before, at least 12 hours before, at least 14 hours before, at least 16 hours before, at least 18 hours before, at least 20 hours before, at least 22 hours before, 1 day before, at least 2 days before, at least 3 days before, at least 4 days before, at least 5 days before, at least 6 days before, at least 7 days before, at least 8 days before, at least 9 days before or at least 10 days before the introduction of the one or more cells to be grafted.

Alternatively and/or additionally, inflammation is induced after the introduction of the one or more cells to be grafted. For example, but not by way of limitation, inflammation is induced at least 10 minutes after, at least 30 minutes after, at least 1 hour after, at least 2 hours after, at least 4 hours after, at least 6 hours after, at least 8 hours after, at least 10 hours after, at least 12 hours after, at least 14 hours after, at least 16 hours after, at least 18 hours after, at least 20 hours after, at least 22 hours after, 1 day after, at least 2 days after, at least 3 days after, at least 4 days after, at least 5 days after, at least 6 days after, at least 7 days after, at least 8 days after, at least 9 days after or at least 10 days after the introduction of the one or more cells to be grafted.

In certain non-limiting embodiments, the cell, *e.g.,* hepatocyte or kidney cells, transplant recipients may be additionally administered with immunosuppressive agents in order to minimize immune rejection of the grafted cells, *e.g.,* hepatocytes or kidney cells. Immunosuppressive agents can reduce or prevent an adverse immune response in the recipient mammal to the foreign or grafted tissue by inhibiting or suppressing any innate immune system activity, including, but not limited to, T-cell and/or B-cell activity. Administration of immunosuppressive agents may include, but is not limited to, the administration of radiation therapy and/or the administration of immunosuppressive drugs. Examples of suitable immunosuppressive drugs include, but are not limited to, steroids (such as corticosteroids, dexamethasone, and prednisone), Cox-1 and Cox-2 inhibitors, macrolide antibiotics (such as rapamycin and tacrolimus), and other substances that limit, reduce, or suppress B-cell, T-cell, and/or other innate immune activity. In certain embodiments, immunosuppressive agents particularly suitable for use with the present disclosure include those immunosuppressive agents known for use with liver and/or kidney transplantation, including, but not limited to, steroids, cyclosporine, rapamycin, azathioprine, prednisone, and OKT3. In a certain embodiment, hepatocyte graft recipients are administered with the immunosuppressive agent tacrolimus, also called FK506, a calcineurin inhibitor that in turn may inhibit IL-2 production and downstream B-lymphocyte activation.

In certain non-limiting embodiments, a subject is not administered an immunosuppressive agent, for example, an agent set forth above in this paragraph (for example, but not by limitation, where autologous hepatocytes or kidney cells are transplanted).

### 5.2.1 Treatment of Liver Diseases and Disorders

The present disclosure provides methods of treating a subject in need of augmented liver function. In certain embodiments, the present disclosure provides methods of propagating hepatocytes in FALCs or lymph nodes for the purpose of producing ectopic liver tissue, which can be used in a subject for therapeutic benefit, *e.g.,* in a subject with reduced liver function. In certain embodiments, the FALCs are located in the omentum.

A subject in need of augmented liver function is a subject lacking sufficient functional liver to maintain a healthy state, including but not limited to a subject having a fibrotic and/or cirrhotic liver, or a liver damaged by disease, trauma or toxic effect. For example, a healthy state is evidenced by one or more liver function parameter falling within a normal range for the subject. Non-limiting examples of liver function parameters include albumin level, alanine transaminase ("ALT") level, aspartate transaminase ("AST") level, creatinine level, total bilirubin level, direct bilirubin level, phenylalanine level, alanine level, glycine level, valine level, glutamate level, prothrombin time, lactate dehydrogenase and alkaline phosphatase; normal ranges for these levels are known in the art for various subjects, including human subjects. In certain non-limiting embodiments, an elevation of one or more, or two or more, or three or more of said parameters by at least about 25% or by at least about 50% relative to normal levels indicates a need for augmented liver function. Achieving augmented liver function in the subject means moving toward the normal range in at least one liver function parameter, for example, but not by limitation, improving by at least about 10 percent or at least about 20 percent or at least about 30 percent or at least about 40 percent or at least about 50 percent.

In certain non-limiting embodiments, augmented liver function is indicated by a prolongation of survival of a subject, for example by at least about 20% or at least about 30%.

In certain embodiments, the subject in need of augmented liver function can be suffering from a liver disease, a liver disorder, liver failure and/or reduced liver function. Non-limiting examples of liver diseases and/or disorders that can be treated by the methods of the present disclosure include metabolic disorders, Crigler-Najjar syndrome type I, acute liver failure, cirrhosis, hemochromatosis, hyperoxaluria, oxalosis, Wilson's disease, Alpha-1 antitrypsin deficiency, liver cancer, hepatitis (alcoholic and autoimmune), fatty liver disease and non-alcoholic fatty liver disease. Non-limiting examples of diseases and/or disorders related to the biliary system (and indirectly to the liver) that can be treated by the methods of the present disclosure include primary biliary cirrhosis and primary sclerosing cholangitis.

In certain non-limiting embodiments, the present disclosure provides for a method of treating a subject in need of augmented liver function, comprising administering, to the subject, a therapeutically effective amount of hepatocytes, wherein at least one of the administered hepatocytes proliferates in or around a FALC or lymph node and generates an ectopic liver tissue, whereby the liver function of the subject is augmented.

In certain embodiments, the hepatocytes transplanted into the subject can be human hepatocytes, non-human hepatocytes or both. A hepatocyte may be syngeneic or allogeneic to the intended recipient. In non-limiting embodiments, the hepatocyte is autologous to the intended recipient (for example, was harvested and expanded in culture prior to transplant, or was generated from a precursor cell or stem cell of the intended recipient). In certain non-limiting embodiments, a hepatocyte of one species may be transplanted into another species, for example, but not limited to, a human hepatocyte transplanted into a non-human (*e.g*., mouse) host. For example, but not by way of limitation, non-human hepatocytes can be grafted in FALCs of a human recipient.

An effective amount or therapeutically effective amount of hepatocytes to be grafted can comprise between about 10⁴ and 10¹¹ hepatocytes, or between about 10⁵ and 10¹⁰ hepatocytes or an amount found to be effective in resulting in at least one site of ectopic liver tissue in a FALC or lymph node. In certain embodiments, an effective amount of hepatocytes may comprise one or more hepatocytes. In certain embodiments, an amount of about 10⁵ to about 10¹¹, of about 10⁵ to about 10⁹, of about 10⁵ to about 10⁸, of about 10⁵ to about 10⁷, of about 10⁵ to about 10⁶, of about 10⁶ to about 10¹¹, of about 10⁶ to about 10¹⁰, of about 10⁶ to about 10⁹, of about 10⁶ to about 10⁸, of about 10⁶ to about 10⁷, of about 10⁷ to about 10¹¹, of about 10⁷ to about 10¹⁰, of about 10⁷ to about 10⁹ or of about 10⁷ to about 10⁸ hepatocytes can be administered to the subject.

In certain embodiments, the hepatocytes can be administered to a subject more than once to achieve the desired ectopic liver tissue. For example, but not by way of limitation, the hepatocytes can be administered to the subject at least two times, at least three times or at least four times. Alternatively or additionally, the hepatocytes can be grafted to two or more different anatomical regions that contain FALCs or lymph nodes to obtain two or more ectopic liver tissues.

In certain non-limiting embodiments, the present disclosure provides for a method of treating a subject in need of augmented liver function, comprising administering, to the subject, a therapeutically effective amount of hepatocytes, and promoting the proliferation of at least one of the hepatocytes in a FALC of the subject. In certain embodiments, such proliferation can be promoted, for example but not by limitation, by administering the hepatocytes locally to the anatomic region containing the FALC being targeted for ectopic liver tissue formation, and/or by providing at least one agent that promotes the formation of an ectopic liver tissue, as disclosed above. In certain embodiments, the agent can be for example, but not limited to, a plurality of bone marrow-derived cells and/or a plurality of stromal cells. In certain embodiments, the agent can be an activator of the LTβR and/or NIK signaling pathway, *e.g.,* an activator of LTβR or NIK or downstream targets thereof, as described above. In certain embodiments, the agent can be bone marrow-derived cells, stromal cells, an activator of the LTβR and/or NIK signaling pathway or combinations thereof. Analogous methods may be used to generate an ectopic tissue in a lymph node to augment liver function.

In certain non-limiting embodiments, the present disclosure provides for a method of generating an ectopic liver tissue comprising introducing, into a FALC, a hepatocyte, and providing at least one agent that promotes the formation of an ectopic liver tissue, for example as set forth above. For example, but not by way of limitation, a method for treating a subject in need of augmented liver function, comprising (a) administering, to the subject, a therapeutically effective amount of hepatocytes into a FALC of a subject to form an ectopic liver tissue, and (b) administering, to the subject, one or more bone marrowderived cells and/or stromal cells to promote the formation of the ectopic liver, *e.g.,* by promoting the proliferation and/or vascularization of the one or more cells to form the ectopic liver tissue. Analogous methods may be used to generate an ectopic tissue in a lymph node.

In certain non-limiting embodiments, a method for treating a subject in need of augmented liver function, can comprise (a) administering, to the subject, a therapeutically effective amount of hepatocytes into a FALC of a subject to form an ectopic liver tissue, (b) administering, to the subject, an activator of the LTβR and/or NIK signaling pathway to promote the formation of the ectopic liver tissue. Analogous methods may be used to generate an ectopic tissue in a lymph node to augment liver function.

In certain non-limiting embodiments, a method for treating a subject in need of augmented liver function, can comprise (a) administering, to the subject, a therapeutically effective amount of hepatocytes into a FALC of a subject to form an ectopic liver tissue, (b) administering, to the subject, one or more bone marrow-derived cells and/or stromal cells to promote the formation of the ectopic liver, *e.g.,* by promoting the proliferation and/or vascularization of the one or more cells to form the ectopic liver tissue, and (c) administering an activator of the LTβR and/or NIK signaling pathway. Analogous methods may be used to generate an ectopic tissue in a lymph node to augment liver function.

In certain embodiments, the method for treating a subject in need of augmented liver function, can further include inducing inflammation in the subject. For example, but not by way of limitation, inflammation can be induced by the administration of an agent that promotes inflammation to the subject, as disclosed above. In certain embodiments, inflammation is induced prior to the introduction of the one or more hepatocytes to be grafted, as described above.

In certain embodiments, the disclosed method is performed *in vivo,* as described above. In certain embodiments, it is performed *in vitro*/*ex vivo,* for example as a cultured tissue explant. In certain non-limiting embodiments, the presently disclosed subject matter provides for liver tissue produced in such an *in vitro*/*ex vivo* method, for example for later transplant into a subject needing augmented liver function.

### 5.2.2 Treatment of Kidney Diseases and Disorders

The present disclosure provides methods of treating a subject in need of augmented kidney function. In certain embodiments, the present disclosure provides methods of propagating kidney cells or fragments of kidney tissue in FALCs or lymph nodes for the purpose of producing ectopic kidney tissue, which can be used in a subject for therapeutic benefit, *e.g.,* in a subject with reduced kidney function. In certain embodiments, the FALCs are located in the omentum.

A subject in need of augmented kidney function is a subject lacking sufficient functional kidney to maintain a healthy state, including but not limited to a subject having kidney failure, or a kidney damaged by disease, trauma or toxic effect. For example, but not by way of limitation, a healthy state is evidenced by one or more kidney function parameters falling within a normal range for the subject. Non-limiting examples of kidney function parameters include creatinine level, protein level and albumin level; normal ranges for these levels are known in the art for various subjects, including human subjects. In certain non-limiting embodiments, an elevation of one or more, or two or more, or three of said parameters by at least about 25% or by at least about 50% relative to normal levels indicates a need for augmented kidney function. Achieving augmented kidney function in the subject means moving toward the normal range in at least one kidney function parameter, for example, but not by limitation, improving by at least about 10 percent or at least about 20 percent or at least about 30 percent or at least about 40 percent or at least about 50 percent. In certain embodiments, a reduction in a subject's glomerular filtration rate (GFR) is indicative of reduced kidney function. For example, but not by way of limitation, a reduction of about 10%, about 20%, of about 30%, of about 40% or of about 50% in a subject's GFR is indicative of reduced kidney function. In certain embodiments, the engrafted cells are capable of producing a concentrated urine.

In certain non-limiting embodiments, augmented kidney function is indicated by a prolongation of survival of a subject, for example by at least about 20% or at least about 30%.

In certain embodiments, the subject in need of augmented kidney function can be suffering from a kidney disease, a kidney disorder, kidney failure and/or reduced kidney function. Non-limiting examples of kidney diseases and/or disorders that can be treated by the methods of the present disclosure include acute kidney failure, chronic kidney disease, glomerulonephritis, Lupus, polycystic kidney disease, nephropathy, nephrosis, kidney malformations and kidney cancer. Other non-limiting examples related to kidney diseases are diminished erythropoiesis, lack of activated Vitamin D and atypical nonthyroidal illnesses.

In certain non-limiting embodiments, the present disclosure provides for a method of treating a subject in need of augmented kidney function, comprising administering, to the subject, a therapeutically effective amount of kidney cells, wherein at least one of the administered kidney cells proliferates in or around a FALC or lymph node and generates an ectopic kidney tissue, whereby the kidney function of the subject is augmented.

In certain embodiments, kidney cells or fragments of kidney tissue are grafted in or around a FALC or lymph node to generate ectopic kidney tissue. In certain embodiments, the kidney cells or fragments of kidney tissue transplanted into the subject can be human or non-human cells. Non-limiting examples of non-human kidney cells include non-human primate kidney cells and kidney cells from various non-human animals such as dogs, cats, horses, mice, rats, hamsters, rabbits, swine, etc. In certain embodiments, the kidney cells or fragments of kidney tissue can be syngeneic or allogeneic to the intended recipient. In non-limiting embodiments, the kidney cell and/or kidney tissue fragment is autologous to the intended recipient (for example, was harvested and expanded in culture prior to transplant, or was generated from a precursor cell or stem cell of the intended recipient). In certain embodiments, the kidney cells are fetal kidney cells or tissue, *e.g.,* metanephroi. In certain embodiments, the kidney cells are not metanephroi, *e.g.,* intact metanephroi. In certain embodiments, the kidney cells can be renal progenitor cells. In certain non-limiting embodiments, a kidney cell and/or kidney tissue fragment of one species may be transplanted into another species, for example, but not limited to, a human kidney cell transplanted into a mouse host. For example, but not by way of limitation, non-human kidney cells and/or kidney tissue fragments can be grafted in FALCs of a human recipient.

In certain embodiments, an effective amount of kidney cells may comprise between about 10⁴ and 10¹¹ kidney cells, or between about 10⁵ and 10¹⁰ kidney cells or an amount found to be effective in resulting in at least one site of ectopic kidney tissue in a FALC. In certain embodiments, an effective amount of kidney cells may comprise one or more kidney cells. In certain embodiments, an amount of about 10⁵ to about 10¹¹, of about 10⁵ to about 10⁹, of about 10⁵ to about 10⁸, of about 10⁵ to about 10⁷, of about 10⁵ to about 10⁶, of about 10⁶ to about 10¹¹, of about 10⁶ to about 10¹⁰, of about 10⁶ to about 10⁹, of about 10⁶ to about 10⁸, of about 10⁶ to about 10⁷, of about 10⁷ to about 10¹¹, of about 10⁷ to about 10¹⁰, of about 10⁷ to about 10⁹ or of about 10⁷ to about 10⁸ kidney cells can be administered to the subject.

In certain embodiments, the kidney cells and/or kidney tissue fragments can be administered to a subject more than once to achieve the desired ectopic kidney tissue. For example, but not by way of limitation, the kidney cells and/or kidney tissue fragments can be administered to the subject at least two times, at least three times or at least four times. Alternatively or additionally, the kidney cells and/or kidney tissue fragments can be grafted to two or more different anatomical regions that contain FALCs or lymph nodes to obtain two or more ectopic kidney tissues.

In certain embodiments, the present disclosure further provides for a method of treating a subject in need of augmented kidney function, comprising administering, to the subject, a therapeutically effective amount of kidney cells (or a fragment of kidney tissue), and promoting the proliferation of a kidney cell in a fat-associated lymphoid cluster or lymph node of the subject. In certain embodiments, such proliferation may be promoted, for example and not by limitation, by administering the kidney cells locally to the anatomic region containing the FALC or lymph node targeted for ectopic kidney tissue formation, and/or by providing at least one agent that promotes the formation of an ectopic kidney tissue.

In certain embodiments, the agent can be, for example, but not limited to, a plurality of bone marrow-derived cells and/or a plurality of stromal cells or stromal endothelial cells. Alternatively and/or additionally, the agent can be an activator of the LTβR and/or NIK signaling pathway, *e.g.,* an activator of LTβR or NIK or downstream targets thereof. In certain embodiments, the agent can be an activator of the non-canonical NF-κB signaling pathway. In certain embodiments, the agent can be bone marrow-derived cells, stromal cells, an activator of the LTβR and/or NIK signaling pathway, an activator of the non-canonical NF-κB signaling pathway or combinations thereof.

In certain non-limiting embodiments, the present disclosure provides for a method of generating an ectopic kidney tissue comprising introducing, into a FALC, a composition comprising kidney cells, and providing at least one agent that promotes the formation of an ectopic kidney tissue, for example as set forth above. For example, but not by way of limitation, a method for treating a subject in need of augmented kidney function, comprising (a) administering, to the subject, a therapeutically effective amount of kidney cells or kidney tissue fragments into a FALC of a subject to form an ectopic kidney tissue, and (b) administering, to the subject, one or more bone marrow-derived cells and/or stromal cells to promote the formation of the ectopic kidney, *e.g.,* by promoting the proliferation and/or vascularization of the one or more cells to form the ectopic kidney tissue. Analogous methods may be used to generate an ectopic tissue in a lymph node.

In certain non-limiting embodiments, a method for treating a subject in need of augmented kidney function, comprising (a) administering, to the subject, a therapeutically effective amount of kidney cells or kidney tissue fragments into a FALC or lymph node of a subject to form an ectopic kidney tissue, and (b) administering, to the subject, one or more bone marrow-derived cells and/or stromal cells to promote the formation of the ectopic kidney, *e.g.,* by promoting the proliferation and/or vascularization of the one or more cells to form the ectopic kidney tissue. Analogous methods may be used to generate an ectopic tissue in a lymph node to augment kidney function.

In certain non-limiting embodiments, a method for treating a subject in need of augmented kidney function, can comprise (a) administering, to the subject, a therapeutically effective amount of kidney cells or kidney tissue fragments into a FALC or lymph node of a subject to form an ectopic kidney tissue, (b) administering, to the subject, an activator of the LTβR and/or NIK signaling pathway to promote the formation of the ectopic kidney tissue. Analogous methods may be used to generate an ectopic tissue in a lymph node to augment kidney function.

In certain embodiments, the method for treating a subject in need of augmented kidney function, can further include inducing inflammation in the subject. For example, but not by way of limitation, inflammation can be induced by the administration of an agent that promotes inflammation to the subject, as disclosed above. In certain embodiments, inflammation is induced prior to the introduction of the one or more hepatocytes to be grafted, as described above.

In certain embodiments, the disclosed method is performed *in vivo,* as described above. In certain embodiments, it is performed *in vitro*/*ex vivo,* for example as a cultured tissue explant. In certain non-limiting embodiments, the presently disclosed subject matter provides for kidney tissue produced in such an *in vitro*/*ex vivo* method, for example for later transplant into a subject needing augmented kidney function.

### 5.3 COMPOSITIONS AND KITS

The presently disclosed subject matter provides compositions for generating ectopic tissue. The present disclosure provides compositions for generating ectopic tissue that include one or more cells and one or more agents that can be used to promote the formation of an ectopic tissue from the one or more cells.

In certain embodiments, the one or more cells present in the composition comprise hepatocytes. In certain embodiments, the one or more cells present in the composition comprise kidney cells. In certain embodiments, hepatocytes or kidney cells may be prepared for transplantation, and/or transplantation procedures may be carried out using methodology described in, United States Patent No. 9,125,891.

The cells suitable for use in the present disclosure can be derived from any suitable source. For example, but not by way of limitation, the cells can be derived from an autologous source. In certain embodiments, the cells can be derived from the subject to be implanted with the cells. In certain embodiments, the cells can be derived from a heterologous source. In certain embodiments, the cells can be derived an individual different from the subject to be implanted with the cells. In certain embodiments, the cells, e.g., hepatocytes or kidney cells, can also be generated from stem cells derived from various sources that are then differentiated into the relevant cell type. In certain embodiments, cells can be cultured for a period of time under various conditions to induce certain phenotypes before use in the presently disclosed compositions and/or methods.

In certain embodiments, the number of cells contained within a composition disclosed herein can vary. In certain embodiments, a composition can include at least about 1, about 2, about 3, about 4, about 5, about 10, about 15, about 20, about 25, about 30, about 35, about 50, about 100, about 150, about 200, about 300, about 400, about 500, about 1000, about 10,000, about 100,000 or about 1,000,000 cells, *e.g.,* hepatocytes or kidney cells. In certain embodiments, a composition can include from about 10⁴ to about 10¹¹ cells, *e.g.,* hepatocytes or kidney cells.

In certain embodiments, the present disclosure provides for a composition for generating an ectopic liver or treating a subject that needs augmented liver function. In certain embodiments, the composition comprises a plurality of hepatocytes and one or more of the following agents that promote the formation of an ectopic liver. In certain embodiments, the agent can be one or more cells that are not hepatocytes. For example, but not by way of limitation, the one or more agents can include a plurality of bone marrow-derived cells and/or a plurality of stromal cells. In certain embodiments, the bone marrow-derived cells can be hematopoietic stem cells. In certain embodiments, the stromal cells can be fibroblast or fibroblast-like cells, such as fibroblastic reticular cells (FRCs), folicular dendritic cells (FDCs), lymphatic endothelial cells (LECs), blood endothelial cells (BECs), alpha-7 integrin pericytes (AIPs) and double negative cells (DNCs).

In certain embodiments, the present disclosure provides for a composition for generating an ectopic kidney or treating a subject that needs augmented kidney function. In certain non-limiting embodiments, the present disclosure provides for a composition for generating an ectopic kidney comprising a plurality of kidney cells (or a fragment of kidney tissue) and one or more of the agents that promote the formation of an ectopic kidney. In certain embodiments, the agent can be one or more cells that are not kidney cells. For example, but not by way of limitation, the one or more agents can include a plurality of bone marrow-derived cells and a plurality of stromal cells. In certain embodiments, the bone marrow-derived cells can be hematopoietic stem cells. In certain embodiments, the stromal cells can be fibroblast or fibroblast-like cells, such as fibroblastic reticular cells (FRCs), folicular dendritic cells (FDCs), lymphatic endothelial cells (LECs), blood endothelial cells (BECs), alpha-7 integrin pericytes (AIPs) and double negative cells (DNCs).

In certain embodiments, the composition can further include an activator of the LTβR and/or NIK signaling pathway. Non-limiting examples of activators of the LTβR and/or NIK signaling pathway are discussed above. In certain embodiments, the composition can further include an activator of the non-canonical NF-κB signaling pathway. For example, but not by way of limitation, a composition of the present disclosure can include a plurality of cells, *e.g.,* kidney cells (or a fragment of kidney tissue) or hepatocytes, and a therapeutically effective amount of an activator of the LTβR and/or NIK signaling pathway.

In certain embodiments, the present disclosure provides a composition that can include (a) a plurality of cells, *e.g.,* kidney cells (or a fragment of kidney tissue) or hepatocytes, (b) a plurality of bone marrow-derived cells and a plurality of stromal cells and (c) a therapeutically effective amount of an activator of the LTβR and/or NIK signaling pathway.

In certain embodiments, the composition can optionally comprise a synthetic culture medium and/or a scaffold. In certain embodiments, the medium and/or scaffold can include, but not limited to, peptides, proteins, carbohydrates, matrigel, hyaluronic acid, collagen, fibrin, fibrinogen, fibronectin, polyorthoester, polyvinyl alcohol, polyamide, polycarbonate, agarose, alginate, poly(ethylene) glycol, polylactic acid, polyglycolic acid, polycaprolactone, polyvinyl pyrrolidone, a marine adhesive protein, cyanoacrylate, polymeric hydrogel, analogs or a combination thereof.

In certain embodiments, the composition may optionally comprise an antibiotic. Non-limiting examples of antibiotics include, but are not limited to, penicillin, e.g., penicillin and amoxicillin; cephalosporins, *e.g.,* cephalexin; sulfonamides, *e.g.,* cotrimoxazole and trimethoprim; macrolides, *e.g.,* erythromycin, clarithromycin and azithromycin; fluoroquinolones, *e.g.,* ciprofloxacin, levofloxacin and ofloxacin; tetracyclines, *e.g.,* tetracycline and doxycycline; and aminoglycosides, *e.g.,* gentamicin and tobramycin.

The present disclosure further provides compositions that include ectopic tissue produced by the methods disclosed above. For example, but not by way of limitation, a composition of the present disclosure can include a human liver tissue produced in a non-human host, as disclosed above. In certain embodiments, the compositions can optionally comprise a synthetic culture medium and/or scaffold. In certain embodiments, the compositions can optionally comprise an antibiotic.

The present disclosure further provides kits that include one or more of the compositions disclosed herein. In certain embodiments, if the kit includes one or more compositions, each composition can be provided in its own container in the kit. In certain embodiments, a kit of the present disclosure further provides instructions for using the kit, *e.g.,* instructions for administering the composition present in the kit. In certain embodiments, a kit of this disclosure can further include one or more components such as instructions for use, devices and additional reagents, and components, such as tubes, containers and syringes for performing the methods disclosed above.

The following Examples are offered to more fully illustrate the disclosure, but are not to be construed as limiting the scope thereof.

### 6. EXAMPLE 1: BIOENGINEERING A LIVER IN SECONDARY LYMPHOID TISSUE

### 6.1. Generating auxiliary liver in lymph node (LN) and milky spots (FALCs)

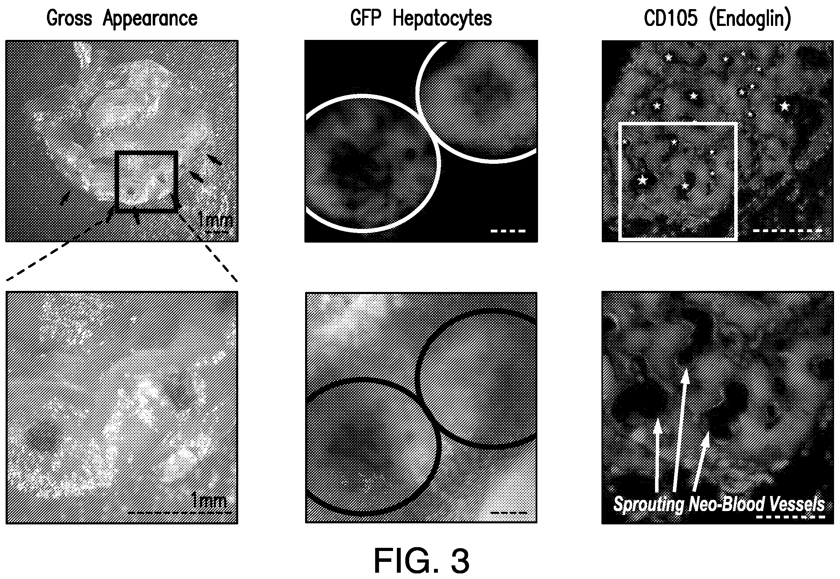

In the quest to identify new sites for hepatocyte transplantation as an alternative to whole organ transplant, an unusual observation was made that hepatocytes could form ectopic nodules *de novo* after intraperitoneal injection (IP) in the Fah^{-/-} mouse (a mouse deficient in the tyrosine catabolic enzyme fumarylacetoacetate hydrolase (Fah)), a model of induced liver failure, following removal of 2-(2-nitro-4-trifluoromethylbenzoyl)-1,3-cyclohexanedione ("NTBC") from the drinking water (32). Initially, normal hepatocytes were observed migrating into the lymphatic system to colonize lymph nodes of the Fah^{-/-}mouse (FIGURE 2A), and generating over 70% of the native liver mass ectopically. This was a surprising result, especially considering how lymph nodes are common sites of initial metastasis for a number of cancers. FIGURE 3 depicts Fah^{-/-} mice 3 to 4 weeks after IP transplantation of hepatocytes; dilation of capillary space and sprouting of blood vessels in milky spots.

This led to a hypothesis that lymph nodes could be targeted directly as a site for ectopic transplantation of multiple cell types to restore tissue and organ function. It was established that direct injection of hepatocytes into a single Fah ^{-/-} mouse lymph node (axillary, popliteal or mesenteric) generated enough functional ectopic liver mass to rescue the survival of these mutant mice affected with lethal metabolic disease (33).

More recently, it was demonstrated that this concept of generating an ectopic liver could be translated into large animals after inducing a liver disease (FIGURE 2B). Here, a pig model of liver disease was generated and ectopic liver tissue growth in lymph nodes by performing a portacaval shunt followed by transplantation of hepatocytes in mesenteric lymph nodes. 6 pigs were transplanted with increased additional hepatic injury using partial hepatectomy. The result of this experiment was the demonstration of hepatocyte engraftment in lymph nodes and the generation of hepatic tissue in all transplanted animals (FIGURE 2B) with increased ectopic liver mass. Portacaval shunt is major surgical procedure for patients with serious liver diseases, but is now less commonly used. It reduces 75% of the blood flow to the liver by creating a new connection between blood vessels (portal vein to vena cava) to relieve debilitating portal hypertension. In addition, this dramatic reduction of the blood flow to the liver is known to induce hepatotrophic factors and hepatocyte proliferation (41).

Today, a similar but preferred minimally-invasive procedure is Transjugular Intrahepatic Portosystemic Shunting (TIPS) to initiate new connections between portal vein to hepatic vein in cirrhotic patients with critical portal hypertension. Because of these promising results in the pig model and its potential translational outcomes for patients after TIPS, how and why the lymphatic microenvironment is such a good host for transplanted cells was to be determined.

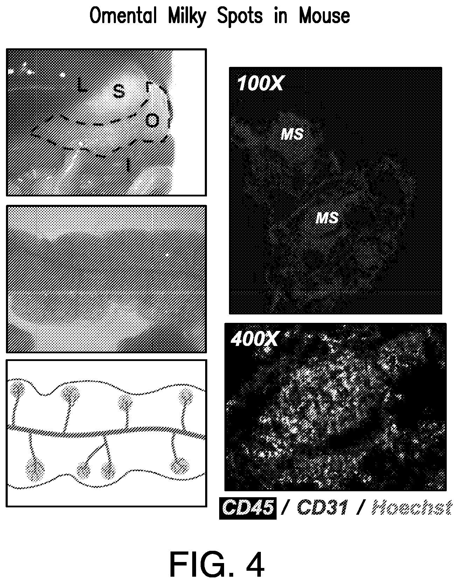

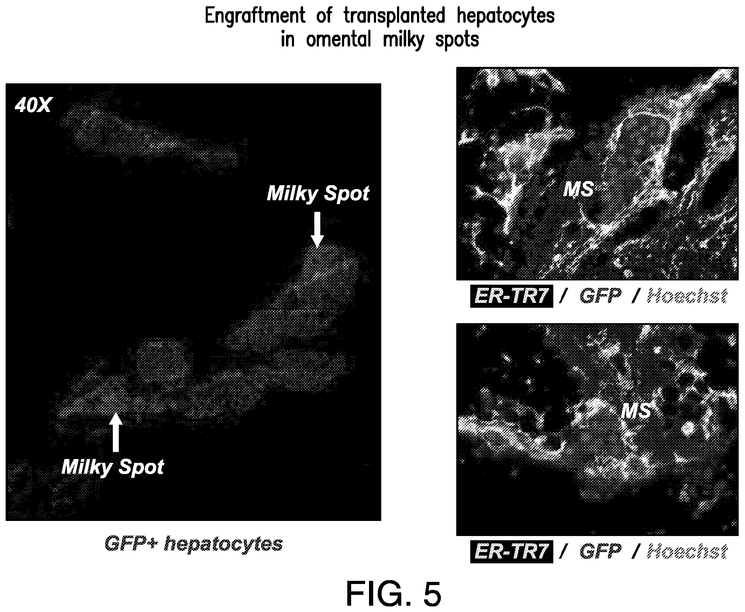

Hepatocytes also engraft into milky spots. When the hepatocyte engraftment site in the Fah^{-/-} mouse after IP injection was carefully analyzed, it was found that, in addition to lymph nodes, hepatocytes also migrated to the omental milky spots, generating hepatic nodules (FIGURE 2C-D, FIGURES 3-5). Note, as shown in FIGURE 3, the dilation of the capillary space and sprouting of blood vessels (*e.g.*, lower right panel) in milky spots. Milky spots are small "milky" colored areas of lymphoid tissue (Fat-Associated Lymhoid Clusters or "FALCs") found mostly in the greater omentum (FIGURE 2C). These aggregates of hematopoietic cells (lymphoid cells and macrophages) and stromal cells are known as secondary lymphoid organs (42, 43), similar to lymph nodes. Not surprisingly, milky spots have also been identified as an early target in peritoneal carcinomatosis, particularly with metastatic ovarian cancer.

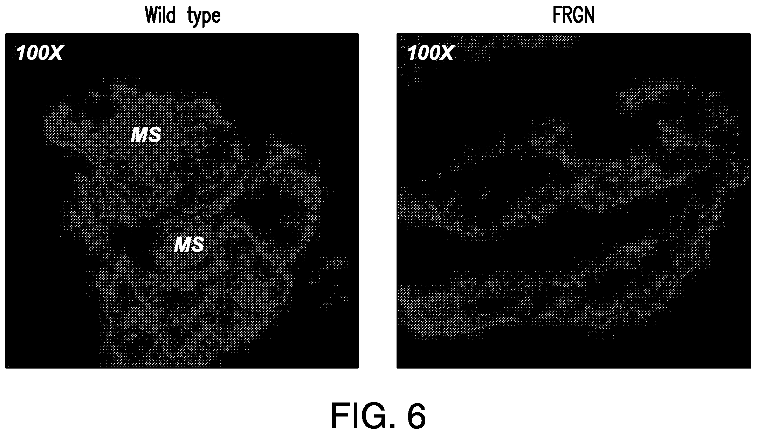

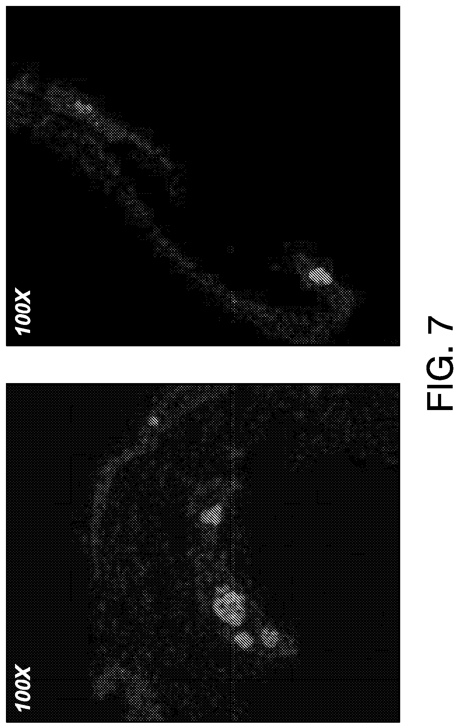

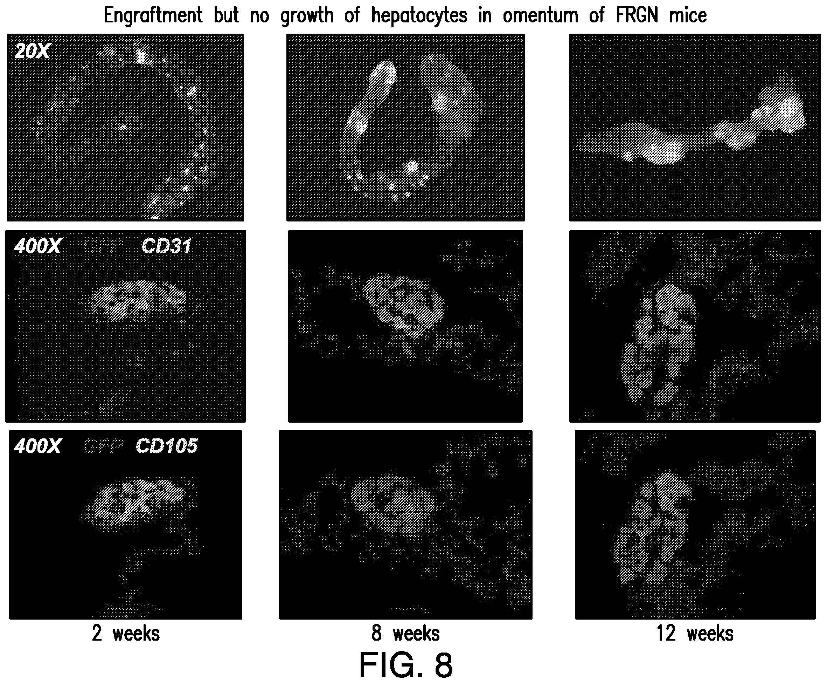

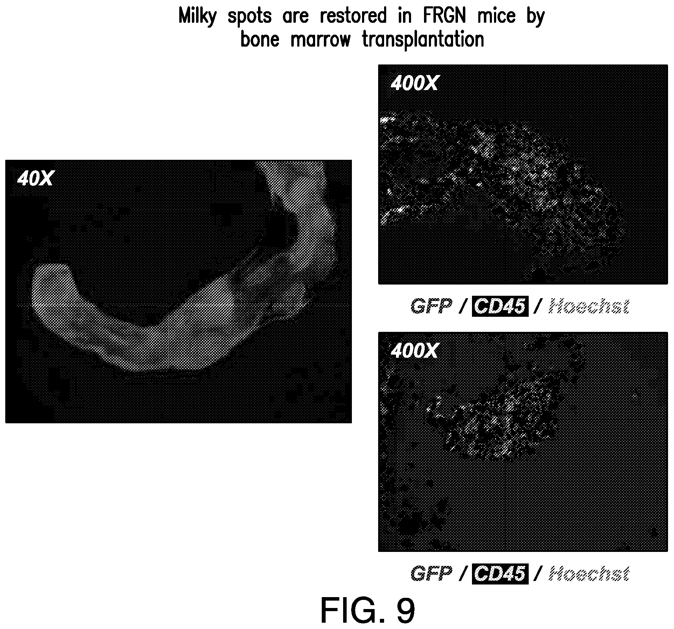

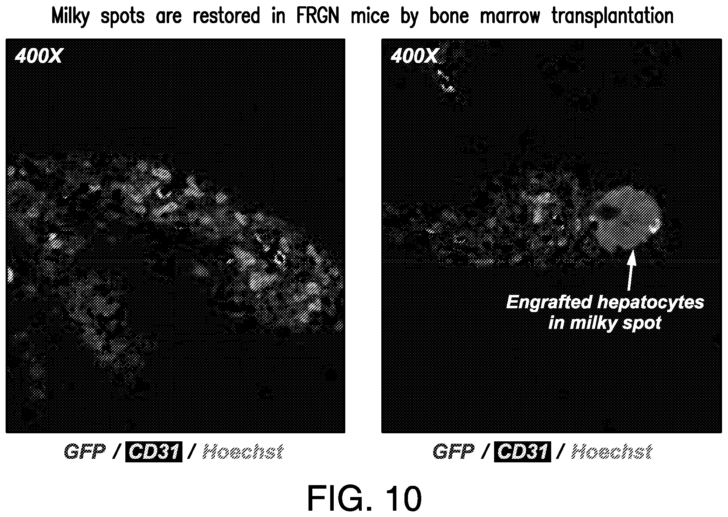

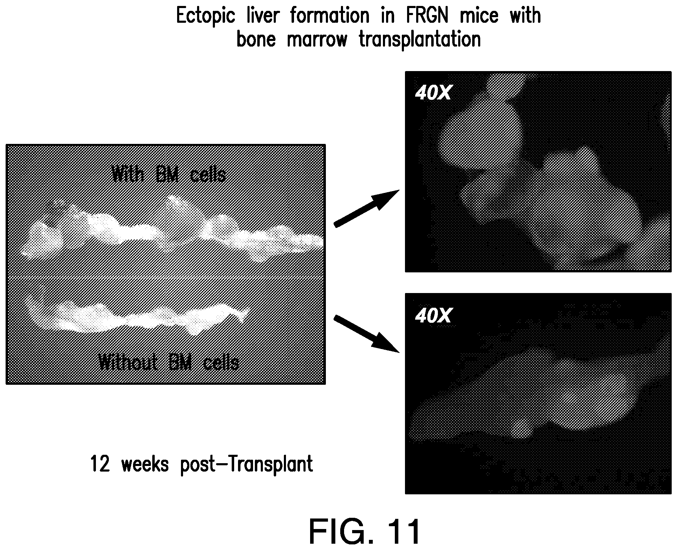

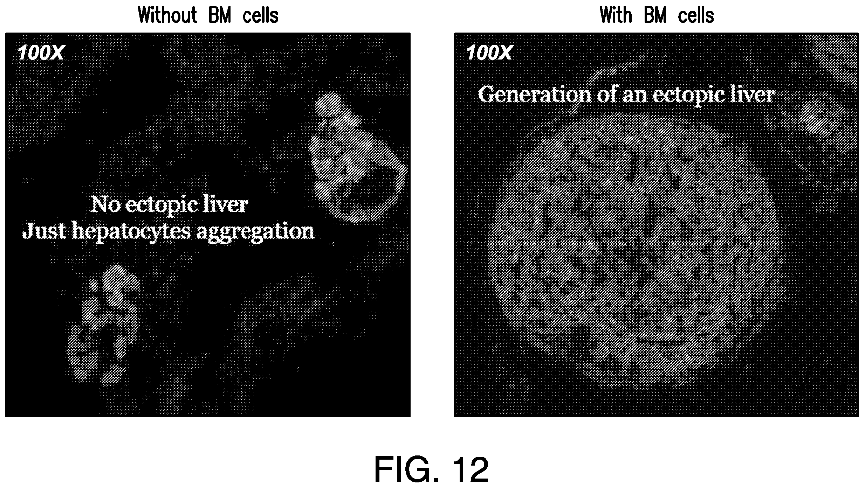

To confirm that ectopic liver formation required a lymphoid tissue environment, IP transplantation was performed in Fah^{-/-} NOD mice further carrying mutations in recombinase activating gene-2 (RAG2) and common cytokine receptor gamma chain (gamma c) ("FRGN" mice); such mice are completely alymphoid (44). As shown in FIGURE 6, omental milky spots, and FALCs, are lacking in FRGN mice. IP transplantation of hepatocytes resulted in poor engraftment in the omentum of FRGN mice relative to wild-type control (FIGURE 7) and growth of engrafted cells was not observed (FIGURE 8). Interestingly, the capacity to form milky spots and FALCs were restored in FRGN mice by bone marrow transplantation (FIGURES 9 and 10) and those milky spots and FALCs could support ectopic liver formation (FIGURES 11 and 12).

### 6.2. A molecular mechanism for ectopic liver development

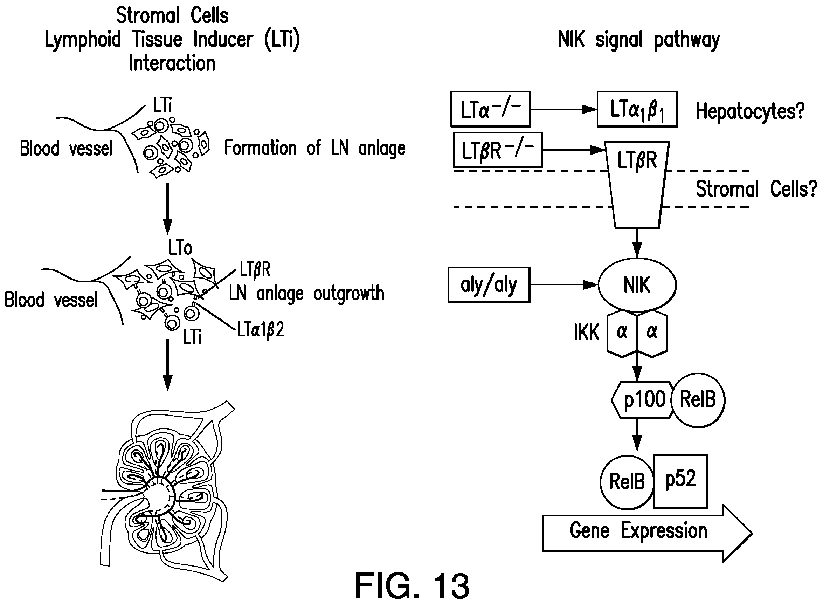

It was then asked whether lymph nodes are required for hepatocyte engraftment and Fah^{-/-} survival. It was reasoned that hepatocytes are colonizing milky spots and lymph nodes, and wanted to know if Fah^{-/-} mice lacking functional lymph nodes could generate enough ectopic liver mass in milky spots to survive. To test this hypothesis, lymphoblastic mice (aly/aly) were crossed with mice that undergo inducible liver failure (Fah^{-/-}). Aly/aly mice lack lymph nodes, Peyer's patches and have a disorganized spleen due to a point mutation in the NF-κB inducing kinase (NIK) that disrupts non-canonical NF-κB signaling in cells (FIGURE 13). Without non-canonical NF-κB signaling, lymph node organogenesis does not occur properly during development (no lymph nodes), although lymphatic vessels, and importantly milky spots, are present and functional in these mice (43).

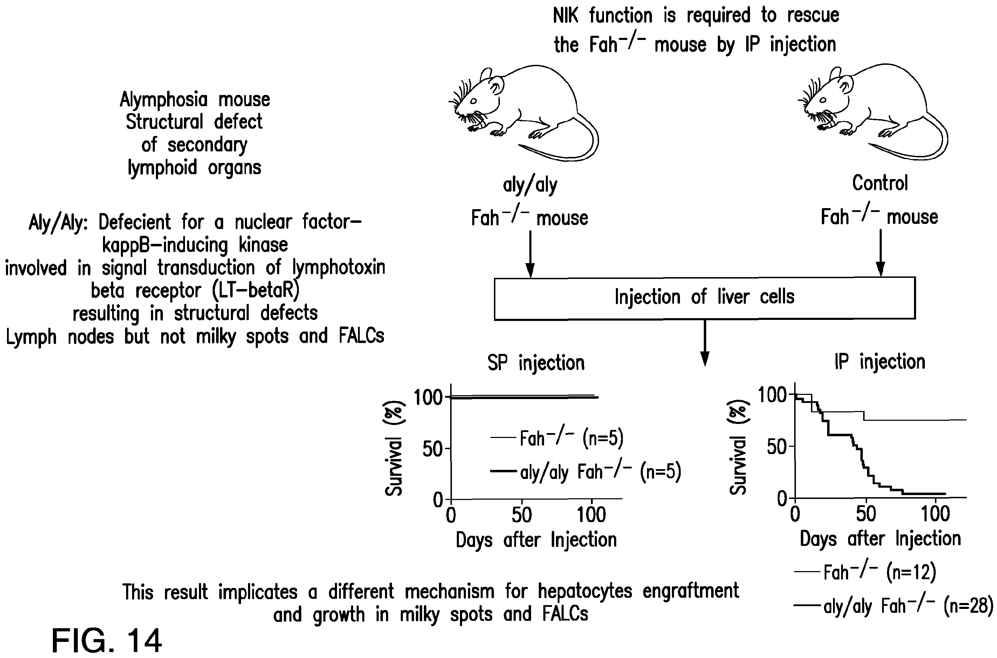

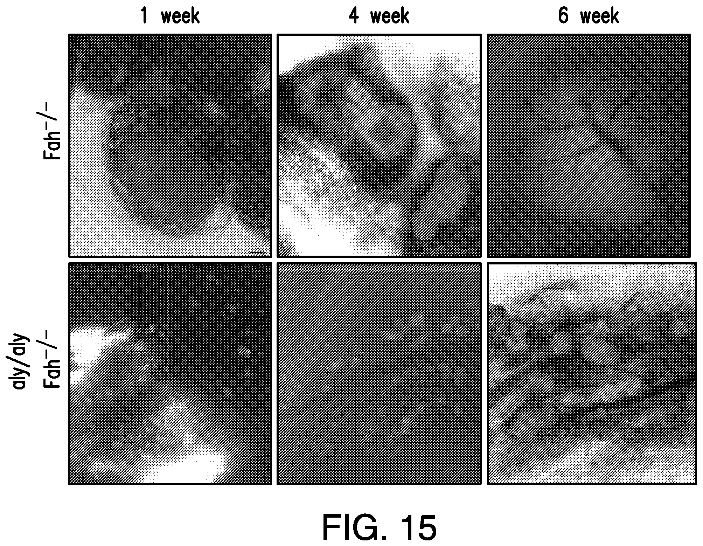

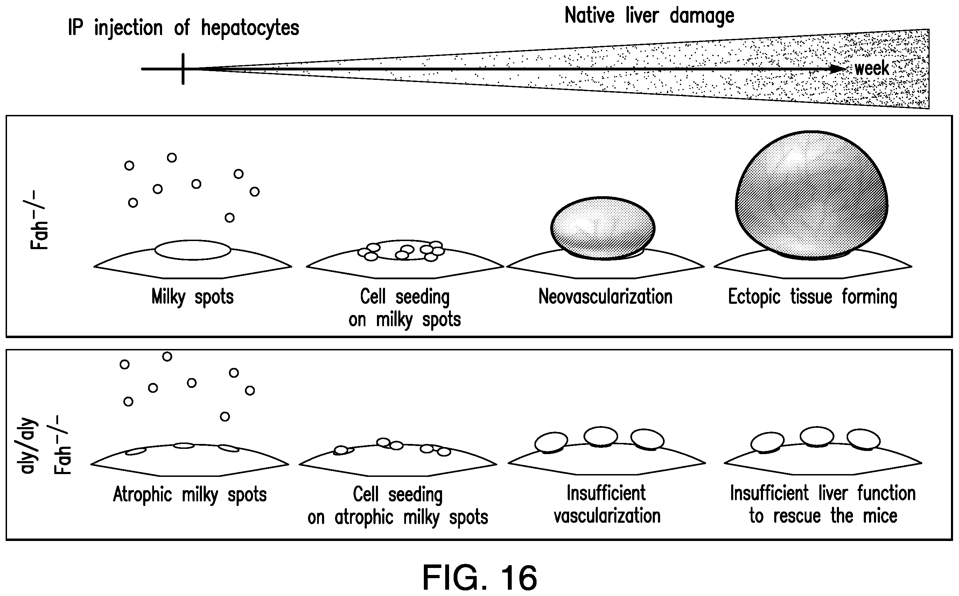

Whether hepatocytes transplanted IP would engraft in aly/aly-Fah^{-/-} mice was first tested (FIGURE 14). It was found that the majority of hepatocytes migrated to the milky spots in the omentum and confirmed that hepatocytes engrafted the omentum of aly/aly-Fah^{-/-} mice similarly to Fah^{-/-} mice (FIGURE 15), but were not able to form large nodules over the course of 6 weeks, and none (n=28) of the transplanted mice were rescued after 12 weeks (the time required to rescue control Fah-/- mice) despite the initial engraftment in the omentum (FIGURE 14, right-most Kaplan Meier survival plot). Upon necropsy, limited vascularization compared to ectopic nodules generated in Fah^{-/-} mice was observed. Hepatocytes were also transplanted into the liver (via splenic injection) of aly/aly-Fah^{-/-} mice, and found that mice were rescued from liver failure (FIGURE 14, left-most Kaplan Meier survival plot). This result is important because it indicates that the lack of peritoneal growth of hepatocytes in aly/aly-Fah^{-/-} mice is unique to this site and is a consequence of the disruption of the NIK pathway due to the aly (NIK) mutation (FIGURE 16). Hepatocytes injected into the liver (splenic injection) presumably replace the dying hepatocytes within the normal liver architecture without need of the NIK pathway.

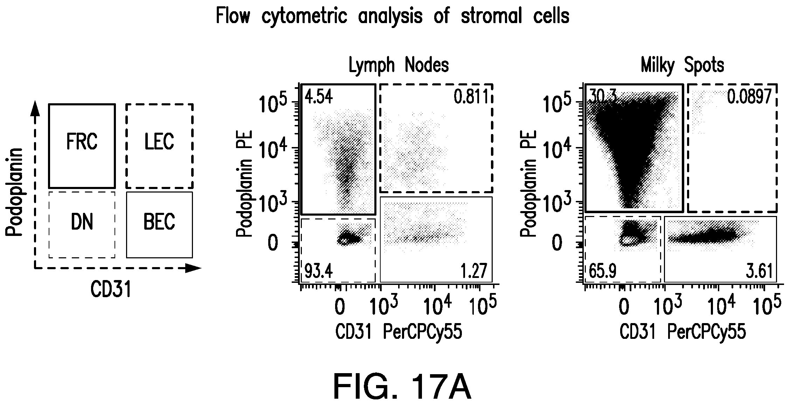

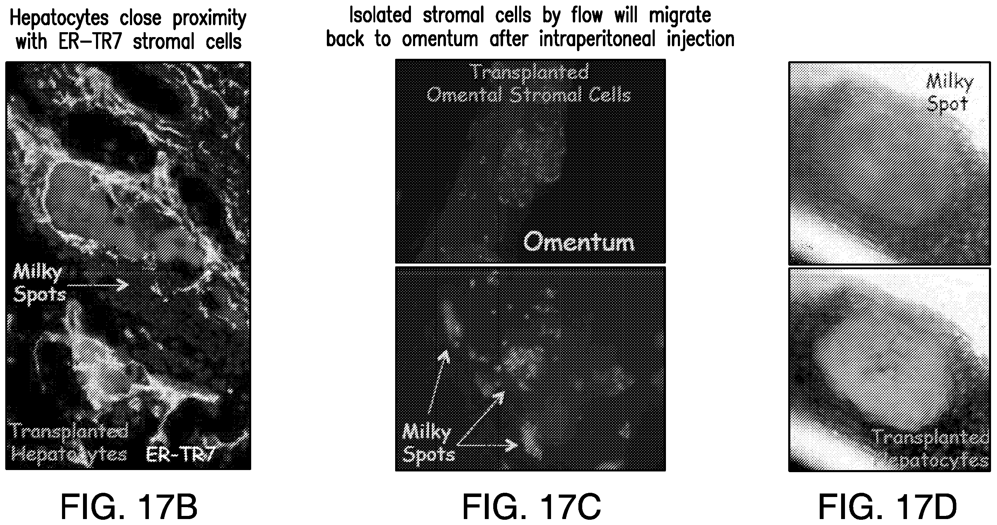

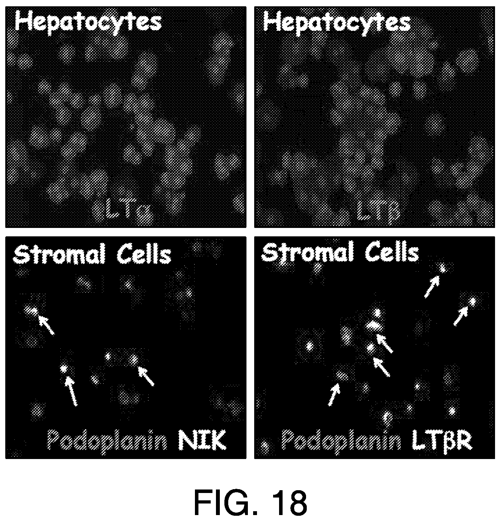

Next, studies were done to characterize the lymphoid environments that can play host to ectopic liver formation. FIGURE 17A shows the distribution of the lymphoid marker podoplanin and the endothelial marker CD31 in lymph nodes as compared to milky spots. As shown in FIGURE 17B, transplanted hepatocytes form close associations with ER-TR7 bearing stromal cells. Interestingly, when omental stromal cells rather than hepatocytes are transplanted via intraperitoneal injection, they, too, migrate back to milky spots in the omentum (FIGURE 17C). As shown in FIGURE 18, the stromal cells were found to express both NIK and the lymphotoxin beta receptor; hepatocytes, in turn, were lymphotoxin alpha (also known as Tumor Necrosis Factor Beta) and beta (also known as Tumor Necrosis Factor C) positive.

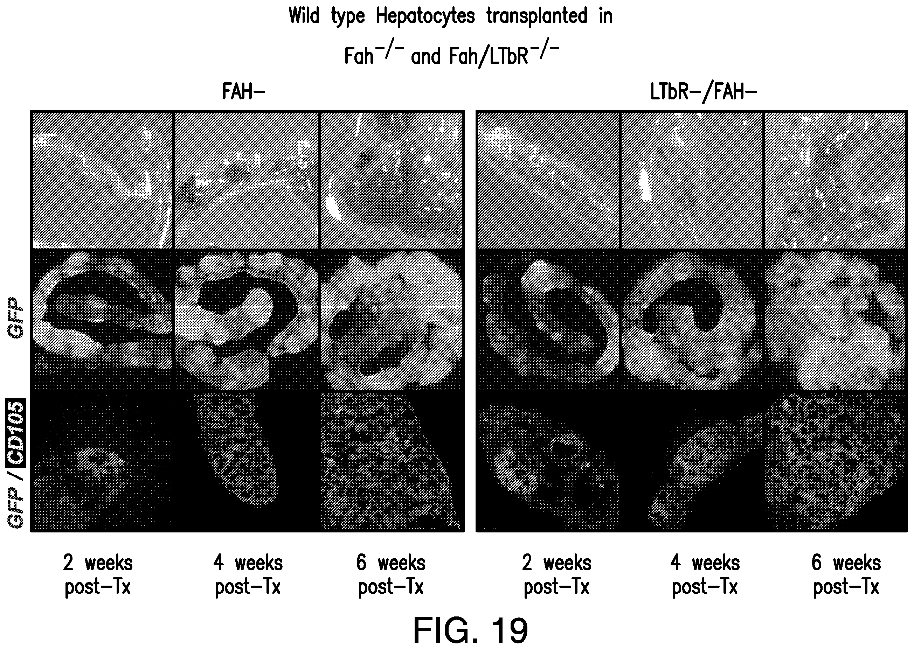

Given the observation that hepatocytes express lymphotoxin beta ("LTb") and stromal cells express lymphotoxin beta receptor ("LTbR") it was of interest to see whether hepatocytes would engraft and grow in milky spots of Fah^{-/-} mice lacking LTbR. The results are shown in FIGURE 19, where growth of hepatocytes in the Fah/LTbR^{-/-}mice was observed to be less than that in Fah^{-/-}, LTbR⁺ mice.

In conclusion, hepatocytes injected IP undergo organogenesis, complete with neo-vascularization to support the ectopic liver mass in Fah^{-/-} mice, and the NIK pathway is a unique pathway necessary for this process to be completed.

In the classic seed and soil hypothesis of cancer by Dr. Stephen Paget (1889), the seed (metastatic cancer) requires the soil (a fertile environment) to succeed. The results described herein demonstrate that the seeds (hepatocytes) require the soil (secondary lymphoid organs) to engraft and generate liver tissue at an ectopic site. Without being bound by any particular theory, the data support a mechanism in which hepatocytes with stromal cells activate the lymphotoxin/NIK pathway to generate an auxiliary liver in milky spots. The data further indicate that regeneration of hepatic tissue, by transplantation of hepatocytes in the liver (splenic route) or in the milky spots (IP route), is mechanistically different. The aly/aly point mutation in the NF-κB inducing kinase (NIK) disrupted ectopic liver development but not native liver repair. NIK has a central function in the non-canonical NF-κB signaling for secondary lymphoid organ development. NIK is a member of the mitogen-activating protein 3 (MAP3) kinases, which following receptor ligation, accumulates to detectable levels. This allows NIK to phosphorylate and activate the inhibitor of κB Kinase α (IKKα), thus initiating IKK α-mediated phosphorylation of p100 (*see* FIGURE 13).

### 6.3. REFERENCES

1. Starzl, T.E. et al. Orthotopic homotransplantation of the human liver. Ann Surg 168, 392-415 (1968).
2. Reuben, A. Alcohol and the liver. Curr Opin Gastroenterol 23, 283-291 (2007).
3. Perkins, J.D. et al. Should liver transplantation in patients with model for end-stage liver disease scores <or= 14 be avoided? A decision analysis approach. Liver Transpl 15, 242-254 (2009).
4. Volk, M.L., Hernandez, J.C., Lok, A.S. & Marrero, J.A. Modified Charlson comorbidity index for predicting survival after liver transplantation. Liver Transpl 13, 1515-1520 (2007).
5. Lipshutz, G.S. & Busuttil, R.W. Liver transplantation in those of advancing age: the case for transplantation. Liver Transpl 13, 1355-1357 (2007).
6. Strom, S.C., Chowdhury, J.R. & Fox, I.J. Hepatocyte transplantation for the treatment of human disease. Semin Liver Dis 19, 39-48 (1999).
7. Rhim, J.A., Sandgren, E.P., Degen, J.L., Palmiter, R.D. & Brinster, R.L. Replacement of diseased mouse liver by hepatic cell transplantation. Science (New York, N.Y.) 263, 1149-1152 (1994).
8. Weglarz, T.C., Degen, J.L. & Sandgren, E.P. Hepatocyte transplantation into diseased mouse liver. Kinetics of parenchymal repopulation and identification of the proliferative capacity of tetraploid and octaploid hepatocytes. Am J Pathol 157, 1963-1974 (2000).
9. Braun, K.M. & Sandgren, E.P. Cellular origin of regenerating parenchyma in a mouse model of severe hepatic injury. Am J Pathol 157, 561-569 (2000).
10. Grompe, M. et al. Pharmacological correction of neonatal lethal hepatic dysfunction in a murine model of hereditary tyrosinaemia type I. Nat Genet 10, 453-460 (1995).
11. Overturf, K., al-Dhalimy, M., Ou, C.N., Finegold, M. & Grompe, M. Serial transplantation reveals the stem-cell-like regenerative potential of adult mouse hepatocytes. The American journal of pathology 151, 1273-1280 (1997).
12. Lagasse, E. et al. Purified hematopoietic stem cells can differentiate into hepatocytes in vivo. Nat Med 6, 1229-1234 (2000).
13. Grompe, M. Principles of therapeutic liver repopulation. J Inherit Metab Dis 29, 421-425 (2006).
14. Grompe, M., Overturf, K., al-Dhalimy, M. & Finegold, M. Therapeutic trials in the murine model of hereditary tyrosinaemia type I: a progress report. J Inherit Metab Dis 21, 518-531 (1998).
15. Overturf, K. et al. Hepatocytes corrected by gene therapy are selected in vivo in a murine model of hereditary tyrosinaemia type I. Nat Genet 12, 266-273 (1996).
16. Fox, I.J. et al. Treatment of the Crigler-Najjar syndrome type I with hepatocyte transplantation. N Engl J Med 338, 1422-1426 (1998).
17. Nishikawa, T. et al. Resetting the transcription factor network reverses terminal chronic hepatic failure. The Journal of clinical investigation 125, 1533-1544 (2015).
18. Tretbar, L.L., Beven, E.G. & Hermann, R.E. Homotransplantation of an auxiliary dog liver into the pelvis; effect of protacaval shunt in the prevention of liver atrophy. Surgical forum 16, 219-221 (1965).
19. Marchioro, T.L., Porter, K.A., Dickinson, T.C., Faris, T.D. & Starzi, T.E. Physiologic Requirements For Auxiliary Liver Homotransplantation. Surgery, gynecology & obstetrics 121, 17-31 (1965).
20. Van Thiel, D.H., Makowka, L. & Starzl, T.E. Liver transplantation: where it's been and where it's going. Gastroenterology clinics of North America 17, 1-18 (1988).
21. Moritz, M.J. et al. Heterotopic liver transplantation for fulminant hepatic failure--a bridge to recovery. Transplantation 50, 524-526 (1990).
22. Boudjema, K. et al. Temporary auxiliary liver transplantation for subacute liver failure in a child. Lancet (London, England) 342, 778-779 (1993).
23. Terpstra, O.T., Reuvers, C.B. & Schalm, S.W. Auxiliary heterotopic liver transplantation. Transplantation 45, 1003-1007 (1988).
24. Metselaar, H.J. et al. Recovery of failing liver after auxiliary heterotopic transplantation. Lancet (London, England) 335, 1156-1157 (1990).
25. Faraj, W. et al. Auxiliary liver transplantation for acute liver failure in children. Annals of surgery 251, 351-356 (2010).
26. Dokmak, S., Elkrief, L. & Belghiti, J. Auxiliary liver transplantation with a small deceased liver graft for cirrhotic liver complicated by hepatocellular carcinoma. Transplant international: official journal of the European Society for Organ Transplantation 26, e102-104 (2013).
27. Ren, W., Zhang, A. & Dong, J. Integrating repopulation and regeneration of the auxiliarily transplanted small liver graft: the solution for organ shortage and immunosuppression. Medical hypotheses 79, 241-245 (2012).
28. McKiernan, P. Liver transplantation and cell therapies for inborn errors of metabolism. Journal of inherited metabolic disease 36, 675-680 (2013).
29. Shanmugam, N.P. et al. Auxiliary liver transplantation: a form of gene therapy in selective metabolic disorders. Journal of clinical and experimental hepatology 1, 118-120 (2011).
30. Rela, M. et al. Auxiliary partial orthotopic liver transplantation for Crigler-Najjar syndrome type I. Annals of surgery 229, 565-569 (1999).
31. Burdelski, M. & Rogiers, X. Liver transplantation in metabolic disorders. Acta gastro-enterologica Belgica 62, 300-305 (1999).
32. Hoppo, T., Komori, J., Manohar, R., Stolz, D.B. & Lagasse, E. Rescue of lethal hepatic failure by hepatized lymph nodes in mice. Gastroenterology 140, 656-666 e652 (2011).
33. Komori, J., Boone, L., DeWard, A., Hoppo, T. & Lagasse, E. The mouse lymph node as an ectopic transplantation site for multiple tissues. Nat Biotechnol 30, 976-983 (2012).
34. DeWard, A.D., Komori, J. & Lagasse, E. Ectopic transplantation sites for cell-based therapy. Current opinion in organ transplantation 19, 169-174 (2014).
35. Francipane, M.G. & Lagasse, E. Maturation of embryonic tissues in a lymph node: a new approach for bioengineering complex organs. Organogenesis, 1-9 (2014).
36. Francipane, M.G. & Lagasse, E. in Stem Cells Translational Medicine In press (2015).
37. Gupta, S. et al. Hepatocytes exhibit superior transgene expression after transplantation into liver and spleen compared with peritoneal cavity or dorsal fat pad: implications for hepatic gene therapy. Hum Gene Ther 5, 959-967 (1994).
38. Jirtle, R.L. & Michalopoulos, G. Effects of partial hepatectomy on transplanted hepatocytes. Cancer Res 42, 3000-3004 (1982).
39. Ohashi, K. et al. Sustained survival of human hepatocytes in mice: A model for in vivo infection with human hepatitis B and hepatitis delta viruses. Nat Med 6, 327-331 (2000).
40. Ohashi, K. et al. Liver tissue engineering at extrahepatic sites in mice as a potential new therapy for genetic liver diseases. Hepatology (Baltimore, Md.) 41, 132-140 (2005).
41. Nordlinger, B. et al. Can hepatocytes proliferate when transplanted into the spleen? Demonstration by autohistoradiography in the rat. European surgical research. Europaische chirurgische Forschung. Recherches chirurgicales europeennes 19, 381-387 (1987).
42. Shah, S. et al. Cellular basis of tissue regeneration by omentum. PloS one 7, e38368 (2012).
43. Rangel-Moreno, J. et al. Omental milky spots develop in the absence of lymphoid tissue-inducer cells and support B and T cell responses to peritoneal antigens. Immunity 30, 731-743 (2009).
44. Mazurier F1, Fontanellas A, Salesse S, Taine L, Landriau S, Moreau-Gaudry F, Reiffers J, Peault B, Di Santo JP, de Verneuil H., A novel immunodeficient mouse model--RAG2 x common cytokine receptor gamma chain double mutants--requiring exogenous cytokine administration for human hematopoietic stem cell engraftment. J Interferon Cytokine Res. 1999 May; 19(5): 533-41.
45. Fabregat I, Moreno-Càceres J, Sánchez A, Dooley S, Dewidar B, Giannelli G, Ten Dijke P; IT-LIVER Consortium., TGF-β signalling and liver disease. FEBS J. 2016 Jun;283(12):2219-32.
46. Rogers S., Lowell J., Hammerman N., and Hammerman M., Transplantation of developing metanephroi into adult rats. Kidney International 1998; 54:27-37.

### 7. EXAMPLE 2: BIOENGINEERING A KIDNEY IN SECONDARY LYMPHOID TISSUE

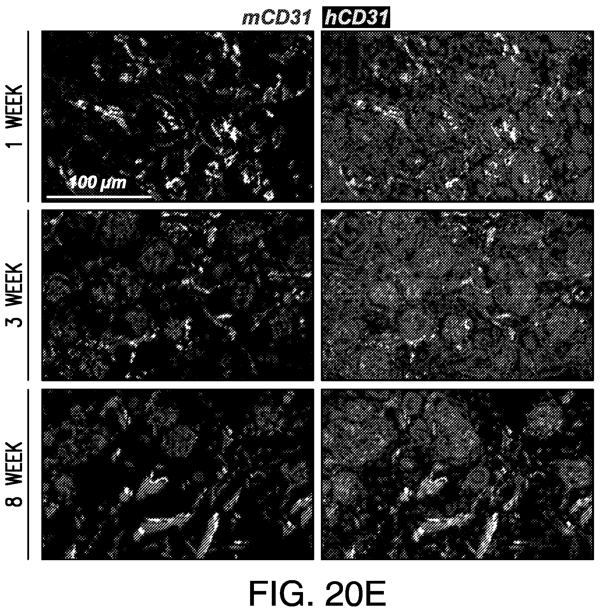

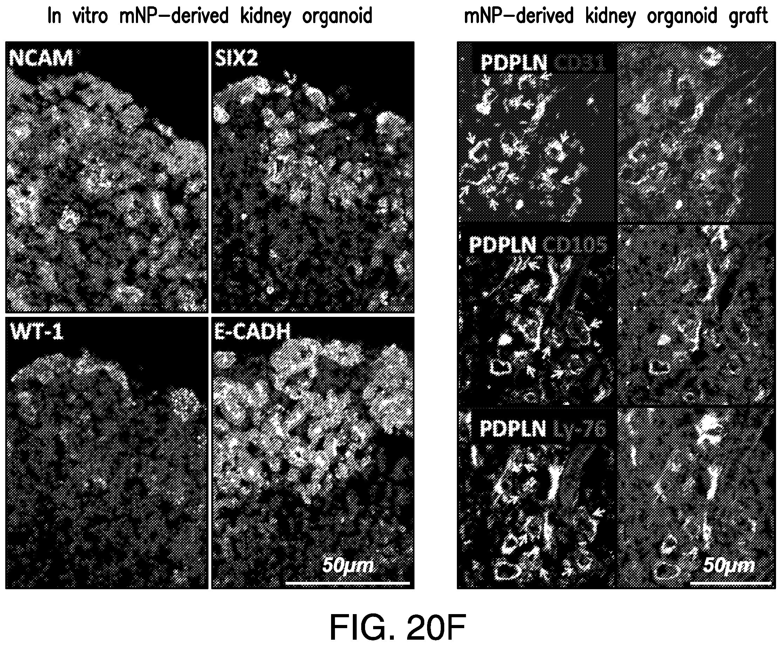

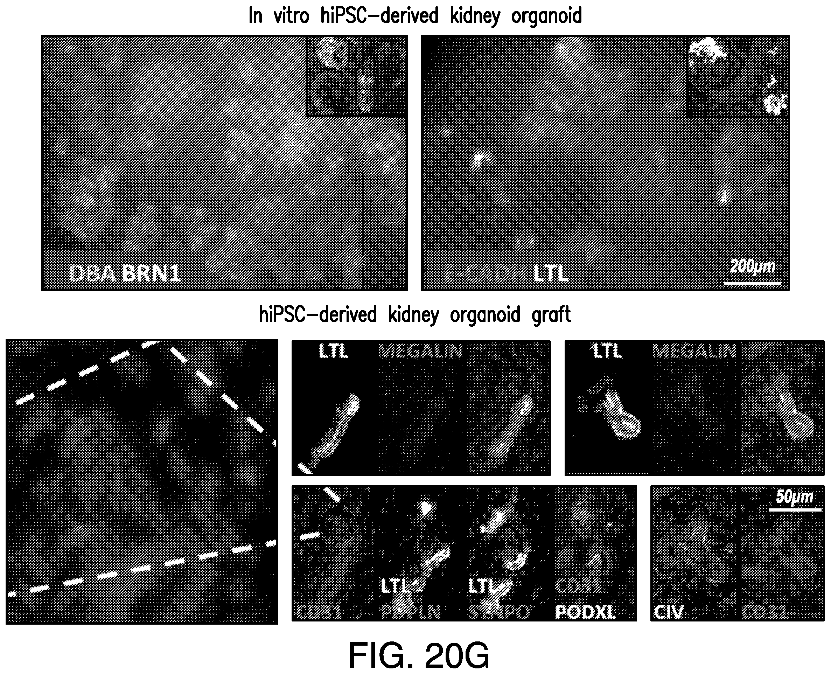

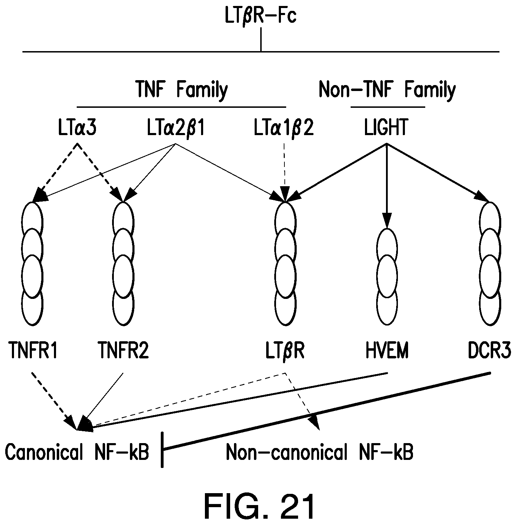

The mouse lymph node (LN), a secondary lymphoid organ (SLO), can support the maturation of mouse metanephroi into nephrons with glomerular and tubular functions, and the LN can also foster the maturation of transplanted human fetal kidney as well as kidney organoid cultures generated from mouse nephron progenitors (NPs) or human induced pluripotent stem cells (hiPSCs) (FIGURE 20). To test whether LTβR signaling, a critical signaling mediator in LN genesis, supported ectopic organogenesis, mice bearing LN kidney grafts were treated with a LTβR-Fc fusion protein. The LTβR-Fc fusion protein antagonizes LTβR-mediated effects by engaging LTβR ligands (LTα and LTβ) (see FIGURE 21). As shown in Figure 22A-B, LTβR-Fc treatment significantly reduced the size of the grafts and their vascularization.

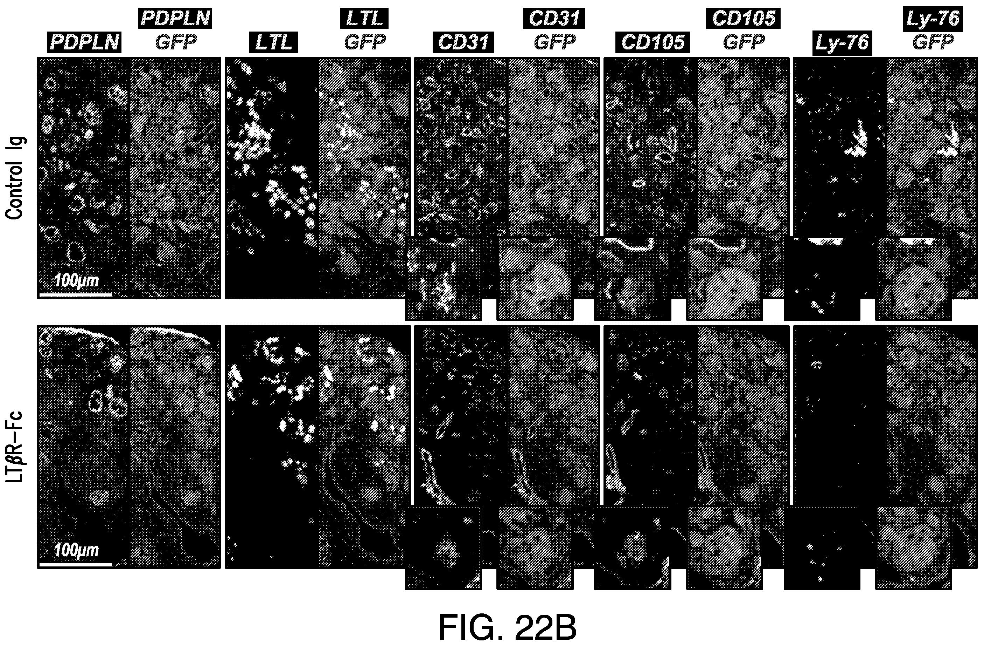

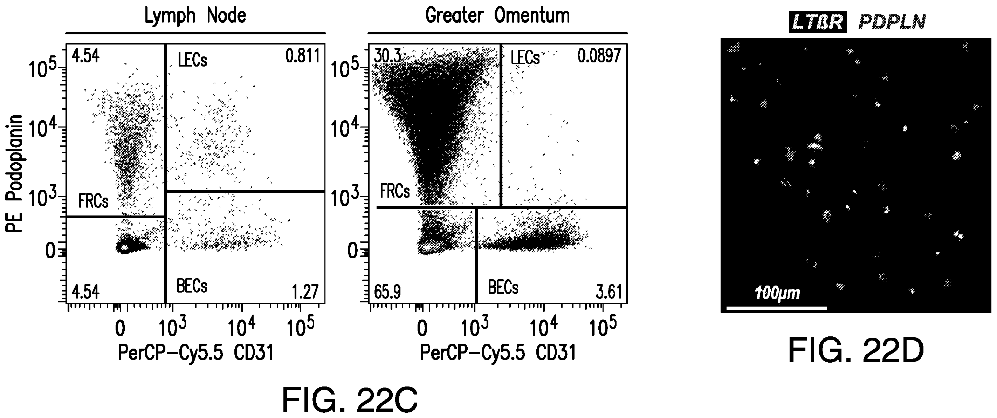

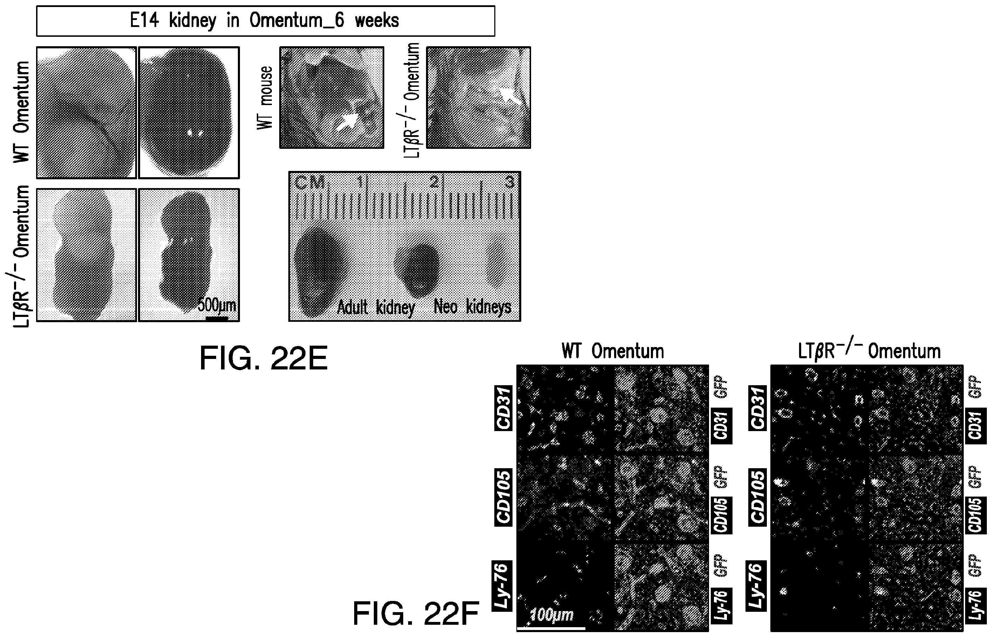

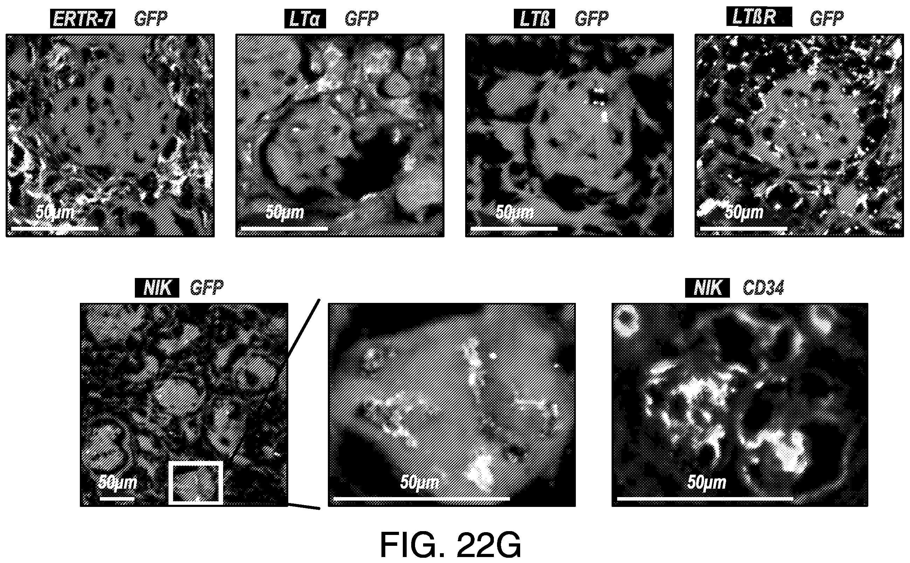

Besides engaging LTβR ligands, LTβR-Fc also engages the non-TNF family member LIGHT, which not only interacts with LTβR, but may also interact with HVEM and Dcr3 receptors (see FIGURE 21). To rule out the possibility that impaired graft vascularization/angiogenesis resulted from LTβR-Fc interference with signaling pathways other than those mediated by LTβR, the outcome of kidney transplantation in an LTβR defective environment was investigated, such as that offered by the LTβR-/- mouse (see FIGURE 22). This approach also allowed the exclusion of the possibility that the observed effects could be due to LTβR/HVEM/Dcr3 inhibition on donor kidney cells or other cell types present in the donor tissue at the time of transplantation, including stromal and hematopoietic cells.

As LTβR-/- mice do not have LNs, the greater omentum was used as an alternative SLO for transplantation. The omentum contains lymphoid aggregates, called milky spots, which promote immunity to peritoneal antigens (see FIGURE 1). Like in the LN, a reticular network of fibroblast reticular cells (FRCs) supports leukocytes in milky spots. When exploring the steady-state stromal composition of omenta as compared to LNs, similar stromal cell flow cytometric profiles were found (FIGURE 22C). Moreover, PDPLN+/LTβR+ and PDPLN-/LTβR+ cell subsets were identified in omental cell suspensions, indicating the existence of rare populations of LT-responsive FRCs and brain endothelial cells (BECs) in the omentum (FIGURE 22D). Growth of embryonic kidney fragments was significantly affected in the LTβR-/- omentum; not only did the grafts grow smaller, but they also were less vascularized, and showed an aberrant morphology when compared to their control counterparts, pinpointing the importance of the LTβR signaling in host stromal cells for successful vascularization/angiogenesis of the ectopic kidney graft (FIGURE 22E-F). In untreated LN kidney grafts, NIK, an LTβR downstream target and central component of the non-canonical NF-κB pathway, was restricted to glomerular endothelial cells (FIGURE 22G). Conversely, LTβR-Fc treatment abrogated NIK expression in these cells. Similarly, NIK expression could not be detected in grafts grown in the LTβR-/- omenta. Without being bound by any particular theory, LTβR might use NIK to propagate the non-canonical NF-κB signaling and promote vascularization/angiogenesis of the transplanted tissue. Further, without being bound by any particular theory, stromal cells residing within secondary lymphoid organs appear to use LTβR-signaling to favor organogenesis, and that stromal endothelial cell-restricted NIK activation may promote graph angiogenesis.

### 8. EXAMPLE 3: INFLAMMATION ENHANCES GROWTH OF GRAFTED HEPTOCYTES

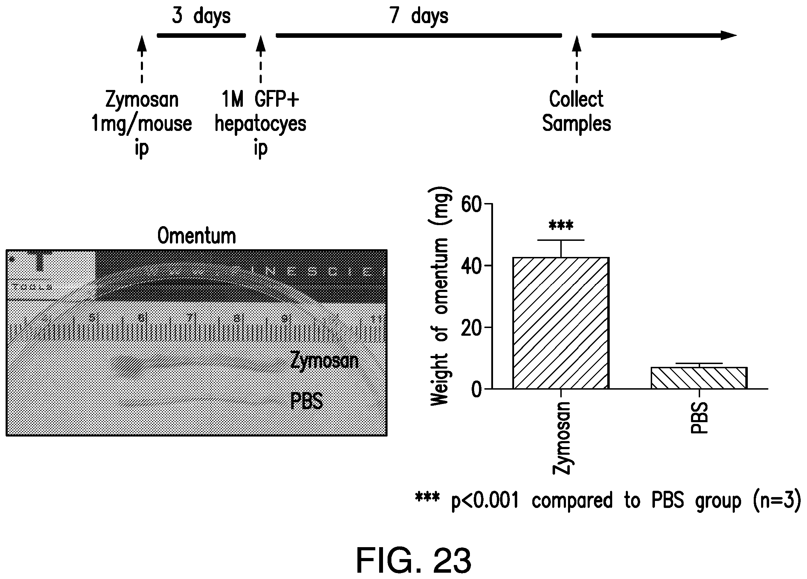

Peritoneal inflammation induces an increase in Fat Associated Lymphoid Clusters (FALCs) number and size (Benezech, C. et al. Inflammation-induced formation of fat-associated lymphoid clusters. Nature immunology 16, 819-828 (2015)). This effect is dependent on TNF expression by myeloid cells and TNFR signaling on stromal cells (Benezech et al. (2015)). To determine if inflammation leads also to increased engraftment of hepatocytes, a sterile peritoneal inflammation driven by Zymosan (a yeast-derived ligand of Toll-like receptor 2) was initiated in wild-type C57bl/6 and control animals (wild-type C57bl/6 injected with PBS) (See FIGURE 23). GFP+ hepatocytes were transplanted 3 days later in both groups of animals and sacrificed after 1 week.

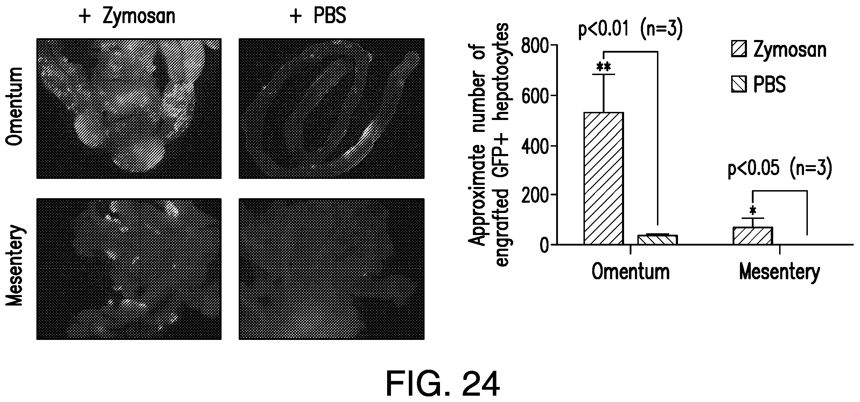

As shown in FIGURE 24, Zymosan-induced inflammation increased dramatically the presence of GFP+ cells, as visualized in tissue and by quantification of GFP+ hepatocytes in omentum and mesenteric fat. Importantly, the engraftment of hepatocytes in FALCs after IP injection was observed in wild type mice indicating that engraftment in FALCs is not dependent on liver injury. How FALC formation is timed with the initiation or resolution of inflammation and whether hepatocyte engraftment will be sustained in the resolution phase remain to be investigated.

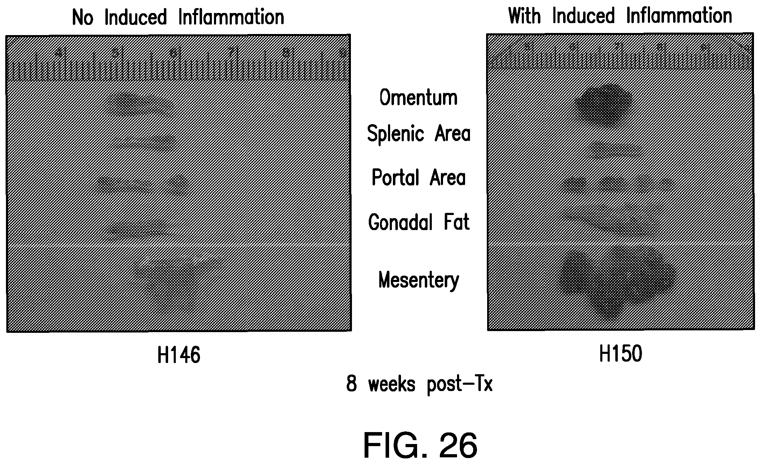

Next, this preconditioning regiment was tested in Fah^{-/-} C57bl/6 mice to determine if it will affect the survival of tyrosinemic mice after inducing a liver disease (off NTBC). Using the same approach as described in FIGURE 23, Fah^{-/-} C57bl/6 mice with or without an induced inflammation were transplanted with wild type hepatocytes followed by inducing liver disease (off NTBC). As shown in FIGURE 25, the animals on the left panel without inflammation were not rescued after two selections (drop in weight = liver disease) while the animal with inflammation was rescued after 8 weeks. Necropsy of the animals without induced inflammation revealed a low/limited hepatic engraftment and liver mass in the FALCs present in omental, splenic, portal, gonadal and mesenteric fat (FIGURE 26). In contrast, the animal with induced inflammation had large masses of hepatic tissue in these locations.

## Claims

1. A composition for use in generating an ectopic tissue in a fat-associated lymphoid cluster of a subject, comprising at least one cell;
wherein the at least one cell is introduced into the fat-associated lymphoid cluster, and wherein the at least one cell comprises a hepatocyte, the ectopic tissue comprises an ectopic liver tissue, and the subject is in need of augmented liver function; or
wherein the at least one cell comprises a kidney cell, a kidney tissue fragment, or a combination thereof, the ectopic tissue comprises an ectopic kidney tissue, and the subject is in need of augmented kidney function.

2. A composition for use in generating an ectopic tissue in a fat-associated lymphoid cluster of a subject, comprising at least one agent;
wherein the at least one agent promotes formation of the ectopic tissue in the fat-associated lymphoid cluster upon administration to the subject;
wherein the composition is for administration with at least one cell that is introduced into the fat-associated lymphoid cluster, wherein the at least one cell comprises a hepatocyte, and the ectopic tissue comprises an ectopic liver tissue, wherein the subject is in need of augmented liver function; or the at least one cell comprises a kidney cell, a kidney tissue fragment, or a combination thereof, and the ectopic tissue comprises an ectopic kidney tissue, wherein the subject is in need of augmented kidney function;
wherein the at least one agent comprises bone marrow-derived cells, stromal cells, fibroblasts, an activator of a lymphotoxin beta receptor (LTβR) signaling pathway, an activator of a NFxB-inducing kinase (NIK) signaling pathway, an inflammation-promoting agent, or a combination thereof.

3. A composition for use in generating an ectopic tissue in a fat-associated lymphoid cluster of a subject, comprising at least one cell;
wherein the at least one cell is introduced into the fat-associated lymphoid cluster, wherein the at least one cell comprises a hepatocyte, and the ectopic tissue comprises an ectopic liver tissue, wherein the subject is in need of augmented liver function; or the at least one cell comprises a kidney cell, a kidney tissue fragment, or a combination thereof, and the ectopic tissue comprises an ectopic kidney tissue, wherein the subject is in need of augmented kidney function; and
wherein the composition is for administration with at least one agent that promotes formation of the ectopic tissue upon administration to the subject;
wherein the at least one agent comprises bone marrow-derived cells, stromal cells, fibroblasts, an activator of a lymphotoxin beta receptor (LTβR) signaling pathway, an activator of a NFxB-inducing kinase (NIK) signaling pathway, an inflammation-promoting agent, or a combination thereof.

4. The composition for use according to claim 2 or claim 3, wherein the at least one agent is administered prior to, after, or concurrently with the administration of the at least one cell.

5. The composition for use according to any one of claims 2-4, wherein the at least one agent comprises at least two of:
(a) cells selected from the bone marrow-derived cells, the stromal cells, the fibroblasts, and combinations thereof;
(b) an activator selected from the activator of the LTβR signaling pathway, the activator of the NIK signaling pathway, and combinations thereof; or
(c) the inflammation-promoting agent.

6. The composition for use according to any one of claims 2-5, wherein the at least one agent comprises the stromal cells, wherein the stromal cells express podoplanin, NIK, LTβR, or a combination thereof.

7. The composition for use according to claim 6, wherein the stromal cells are contacted with the activator of the LTβR signaling pathway, the activator of the NIK signaling pathway, or a combination thereof.

8. The composition for use according to any one of claims 2-7, wherein the at least one agent comprises the activator of the LTβR signaling pathway and the activator of the LTβR signaling pathway comprises an activator of LTβR, or wherein the at least one agent comprises the activator of the NIK signaling pathway and the activator of the NIK signaling pathway comprises an activator of NIK.

9. The composition for use according to any one of claims 2-8, wherein the at least one agent promotes proliferation of the at least one cell, vascularization of the ectopic tissue, or a combination thereof.

10. The composition for use according to any one of claims 2-9, wherein the at least one agent comprises the inflammation-promoting agent, wherein the inflammation-promoting agent is administered to the subject prior to introducing the at least one cell into the fat-associated lymphoid cluster.

11. The composition for use according to any one of claims 1-10, wherein the fat-associated lymphoid cluster is located in an adipose tissue of a pleural cavity, a pericardial cavity, or a peritoneal cavity.

12. The composition for use according to any one of claims 1-10, wherein the fat-associated lymphoid cluster is located in omental fat, mesenteric fat, splenic fat, portal fat, gonadal fat, or a combination thereof.

13. The composition for use according to any one of claims 1-12, wherein the at least one cell is administered locally to an anatomical region of the fat associated lymphoid cluster.

14. The composition for use according to any one of claims 1-13, wherein the at least one cell comprises the hepatocyte, and the ectopic tissue comprises the ectopic liver tissue.

15. The composition for use according to any one of claims 1-13, wherein the at least one cell comprises the kidney cell, the kidney tissue fragment, or the combination thereof, and the ectopic tissue comprises the ectopic kidney tissue.

## Patentansprüche

1. Zusammensetzung zur Verwendung beim Erzeugen eines ektopischen Gewebes in einem fettassoziierten Lymphoid-Cluster eines Individuums, umfassend mindestens eine Zelle;
wobei die mindestens eine Zelle in den fettassoziierten Lymphoid-Cluster eingebracht wird und
wobei die mindestens eine Zelle einen Hepatozyten umfasst, das ektopische Gewebe ein ektopisches Lebergewebe umfasst und das Individuum einer verstärkten Leberfunktion bedarf; oder
wobei die mindestens eine Zelle eine Nierenzelle, ein Nierengewebsfragment oder eine Kombination daraus umfasst, das ektopische Gewebe ein ektopisches Nierengewebe umfasst und das Individuum einer verstärkten Nierenfunktion bedarf.

2. Zusammensetzung zur Verwendung beim Erzeugen eines ektopischen Gewebes in einem fettassoziierten Lymphoid-Cluster eines Individuums, umfassend mindestens einen Wirkstoff;
wobei der mindestens eine Wirkstoff die Bildung des ektopischen Gewebes in dem fettassoziierten Lymphoid-Gewebe bei Verabreichung an das Individuum fördert;
wobei die Zusammensetzung zur Verabreichung mit mindestens einer Zelle, die in den fettassoziierten Lymphoid-Cluster eingebracht wird, dient, wobei die mindestens eine Zelle einen Hepatozyten umfasst und das ektopische Gewebe ein ektopisches Lebergewebe umfasst, wobei das Individuum einer verstärkten Leberfunktion bedarf; oder die mindestens eine Zelle eine Nierenzelle, ein Nierengewebsfragment oder eine Kombination daraus umfasst und das ektopische Gewebe ein ektopisches Nierengewebe umfasst, wobei das Individuum einer verstärkten Nierenfunktion bedarf;
wobei der mindestens eine Wirkstoff von Knochenmark stammende Zellen, Stromazellen, Fibroblasten, einen Aktivator eines Signalwegs des Lymphotoxin-beta-Rezeptors (LTβR), einen Aktivator eines Signalwegs der NFκBinduzierenden Kinase (NIK), einen entzündungsfördernden Wirkstoff oder eine Kombination daraus umfasst.

3. Zusammensetzung zur Verwendung beim Erzeugen eines ektopischen Gewebes in einem fettassoziierten Lymphoid-Cluster eines Individuums, umfassend mindestens eine Zelle;
wobei die mindestens eine Zelle in den fettassoziierten Lymphoid-Cluster eingebracht wird, wobei die mindestens eine Zelle einen Hepatozyten umfasst und das ektopische Gewebe ein ektopisches Lebergewebe umfasst, wobei das Individuum einer verstärkten Leberfunktion bedarf; oder die mindestens eine Zelle eine Nierenzelle, ein Nierengewebsfragment oder eine Kombination daraus umfasst und das ektopische Gewebe ein ektopisches Nierengewebe umfasst, wobei das Individuum einer verstärkten Nierenfunktion bedarf; und
wobei die Zusammensetzung zur Verabreichung mit mindestens einem Wirkstoff dient, der die Bildung des ektopischen Gewebes bei Verabreichung an das Individuum fördert;
wobei der mindestens eine Wirkstoff von Knochenmark stammende Zellen, Stromazellen, Fibroblasten, einen Aktivator eines Signalwegs des Lymphotoxin-beta-Rezeptors (LTβR), einen Aktivator eines Signalwegs der NFκBinduzierenden Kinase (NIK), einen entzündungsfördernden Wirkstoff oder eine Kombination daraus umfasst.

4. Zusammensetzung zur Verwendung nach Anspruch 2 oder Anspruch 3, wobei der mindestens eine Wirkstoff vor, nach oder gleichzeitig mit der Verabreichung der mindestens einen Zelle verabreicht wird.

5. Zusammensetzung zur Verwendung nach einem der Ansprüche 2-4, wobei der mindestens eine Wirkstoff mindestens zwei von Folgendem umfasst:
(a)Zellen, die aus den von Knochenmark stammenden Zellen, den Stromazellen, den Fibroblasten und Kombinationen daraus ausgewählt sind;
(b)einem Aktivator, der aus dem Aktivator des LTβR-Signalwegs, dem Aktivator des NIK-Signalwegs und Kombinationen daraus ausgewählt ist; oder
(c)dem entzündungsfördernden Wirkstoff.

6. Zusammensetzung zur Verwendung nach einem der Ansprüche 2-5, wobei der mindestens eine Wirkstoff die Stromazellen umfasst, wobei die Stromazellen Podoplanin, NIK, LTßR oder eine Kombination daraus exprimieren.

7. Zusammensetzung zur Verwendung nach Anspruch 6, wobei die Stromazellen mit dem Aktivator des LTβR-Signalwegs, dem Aktivator des NIK-Signalwegs oder einer Kombination daraus kontaktiert werden.

8. Zusammensetzung zur Verwendung nach einem der Ansprüche 2-7, wobei der mindestens eine Wirkstoff den Aktivator des LTβR-Signalwegs umfasst und der Aktivator des LTβR-Signalwegs einen Aktivator von LTβR umfasst oder wobei der mindestens eine Wirkstoff den Aktivator des NIK-Signalwegs umfasst und der Aktivator des NIK-Signalwegs einen Aktivator von NIK umfasst.

9. Zusammensetzung zur Verwendung nach einem der Ansprüche 2-8, wobei der mindestens eine Wirkstoff die Proliferation der mindestens einen Zelle, die Vaskularisierung des ektopischen Gewebes oder eine Kombination daraus fördert.

10. Zusammensetzung zur Verwendung nach einem der Ansprüche 2-9, wobei der mindestens eine Wirkstoff den entzündungsfördernden Wirkstoff umfasst, wobei der entzündungsfördernde Wirkstoff dem Individuum vor dem Einbringen der mindestens einen Zelle in den fettassoziierten Lymphoid-Cluster verabreicht wird.

11. Zusammensetzung zur Verwendung nach einem der Ansprüche 1-10, wobei sich der fettassoziierte Lymphoid-Cluster in einem adipösen Gewebe einer Pleurahöhle, einer Perikardhöhle oder einer Peritonealhöhle befindet.

12. Zusammensetzung zur Verwendung nach einem der Ansprüche 1-10, wobei sich der fettassoziierte Lymphoid-Cluster in omentalem Fett, mesenterialem Fett, Milzfett, Portalfett, gonadalem Fett oder einer Kombination daraus befindet.

13. Zusammensetzung zur Verwendung nach einem der Ansprüche 1-12, wobei die mindestens eine Zelle lokal in einen anatomischen Bereich des fettassoziierten Lymphoid-Clusters verabreicht wird.

14. Zusammensetzung zur Verwendung nach einem der Ansprüche 1-13, wobei die mindestens eine Zelle den Hepatozyten umfasst und das ektopische Gewebe das ektopische Lebergewebe umfasst.

15. Zusammensetzung zur Verwendung nach einem der Ansprüche 1-13, wobei die mindestens eine Zelle die Nierenzelle, das Nierengewebsfragment oder die Kombination daraus umfasst und das ektopische Gewebe das ektopische Nierengewebe umfasst.

## Revendications

1. Composition pour une utilisation dans la génération d'un tissu ectopique dans un amas lymphoïde associé à la graisse d'un sujet, comprenant au moins une cellule ;
dans laquelle l'au moins une cellule est introduite dans l'amas lymphoïde associé à la graisse, et
dans laquelle l'au moins une cellule comprend un hépatocyte, le tissu ectopique comprend un tissu hépatique ectopique, et le sujet a besoin d'une fonction hépatique augmentée ; ou
dans laquelle l'au moins une cellule comprend une cellule rénale, un fragment de tissu rénal, ou une combinaison de ceux-ci, le tissu ectopique comprend un tissu rénal ectopique, et le sujet a besoin d'une fonction rénale augmentée.

2. Composition pour une utilisation dans la génération d'un tissu ectopique dans un amas lymphoïde associé à la graisse d'un sujet, comprenant au moins un agent ;
dans laquelle l'au moins un agent favorise la formation du tissu ectopique dans l'amas lymphoïde associé à la graisse lors de l'administration au sujet ;
dans laquelle la composition est destinée à une administration avec au moins une cellule qui est introduite dans l'amas lymphoïde associé à la graisse,
dans laquelle l'au moins une cellule comprend un hépatocyte, et le tissu ectopique comprend un tissu hépatique ectopique, dans laquelle le sujet a besoin d'une fonction hépatique augmentée ; ou l'au moins une cellule comprend une cellule rénale, un fragment de tissu rénal, ou une combinaison de ceux-ci, et le tissu ectopique comprend un tissu rénal ectopique, dans laquelle le sujet a besoin d'une fonction rénale augmentée ;
dans laquelle l'au moins un agent comprend des cellules dérivées de la moelle osseuse, des cellules stromales, des fibroblastes, un activateur d'une voie de signalisation du récepteur de la lymphotoxine bêta (LTβR), un activateur d'une voie de signalisation de la kinase induisant NFκB (NIK), un agent favorisant l'inflammation, ou une combinaison de ceux-ci.

3. Composition pour une utilisation dans la génération d'un tissu ectopique dans un amas lymphoïde associé à la graisse d'un sujet, comprenant au moins une cellule ;
dans laquelle l'au moins une cellule est introduite dans l'amas lymphoïde associé à la graisse, dans laquelle l'au moins une cellule comprend un hépatocyte, et le tissu ectopique comprend un tissu hépatique ectopique, dans laquelle le sujet a besoin d'une fonction hépatique augmentée ; ou l'au moins une cellule comprend une cellule rénale, un fragment de tissu rénal, ou une combinaison de ceux-ci, et le tissu ectopique comprend un tissu rénal ectopique, dans laquelle le sujet a besoin d'une fonction rénale augmentée ; et
dans laquelle la composition est destinée à une administration avec au moins un agent qui favorise la formation du tissu ectopique lors de l'administration au sujet ;
dans laquelle l'au moins un agent comprend des cellules dérivées de la moelle osseuse, des cellules stromales, des fibroblastes, un activateur d'une voie de signalisation du récepteur de la lymphotoxine bêta (LTβR), un activateur d'une voie de signalisation de la kinase induisant NFκB (NIK), un agent favorisant l'inflammation, ou une combinaison de ceux-ci.

4. Composition pour une utilisation selon la revendication 2 ou la revendication 3, dans laquelle l'au moins un agent est administré avant, après, ou simultanément à l'administration de l'au moins une cellule.

5. Composition pour une utilisation selon l'une quelconque des revendications 2 à 4, dans laquelle l'au moins un agent comprend au moins deux parmi :
(a) des cellules sélectionnées parmi les cellules dérivées de la moelle osseuse, les cellules stromales, les fibroblastes, et des combinaisons de ceux-ci ;
(b) un activateur sélectionné parmi l'activateur de la voie de signalisation de LTβR, l'activateur de la voie de signalisation de NIK, et des combinaisons de ceux-ci ; ou
(c) l'agent favorisant l'inflammation.

6. Composition pour une utilisation selon l'une quelconque des revendications 2 à 5, dans laquelle l'au moins un agent comprend les cellules stromales, dans laquelle les cellules stromales expriment la podoplanine, NIK, LTβR, ou une combinaison de ceux-ci.

7. Composition pour une utilisation selon la revendication 6, dans laquelle les cellules stromales sont mises en contact avec l'activateur de la voie de signalisation de LTβR, l'activateur de la voie de signalisation de NIK, ou une combinaison de ceux-ci.

8. Composition pour une utilisation selon l'une quelconque des revendications 2 à 7, dans laquelle l'au moins un agent comprend l'activateur de la voie de signalisation de LTβR et l'activateur de la voie de signalisation de LTβR comprend un activateur de LTβR, ou dans laquelle l'au moins un agent comprend l'activateur de la voie de signalisation de NIK et l'activateur de la voie de signalisation de NIK comprend un activateur de NIK.

9. Composition pour une utilisation selon l'une quelconque des revendications 2 à 8, dans laquelle l'au moins un agent favorise la prolifération de l'au moins une cellule, la vascularisation du tissu ectopique, ou une combinaison de celles-ci.

10. Composition pour une utilisation selon l'une quelconque des revendications 2 à 9, dans laquelle l'au moins un agent comprend l'agent favorisant l'inflammation, lequel agent favorisant l'inflammation est administré au sujet avant l'introduction de l'au moins une cellule dans l'amas lymphoïde associé à la graisse.

11. Composition pour une utilisation selon l'une quelconque des revendications 1 à 10, dans laquelle l'amas lymphoïde associé à la graisse est situé dans un tissu adipeux d'une cavité pleurale, d'une cavité péricardique, ou d'une cavité péritonéale.

12. Composition pour une utilisation selon l'une quelconque des revendications 1 à 10, dans laquelle l'amas lymphoïde associé à la graisse est situé dans la graisse omentale, la graisse mésentérique, la graisse splénique, la graisse portale, la graisse gonadique, ou une combinaison de celles-ci.

13. Composition pour une utilisation selon l'une quelconque des revendications 1 à 12, dans laquelle l'au moins une cellule est administrée localement à une région anatomique de l'amas lymphoïde associé à la graisse.

14. Composition pour une utilisation selon l'une quelconque des revendications 1 à 13, dans laquelle l'au moins une cellule comprend l'hépatocyte, et le tissu ectopique comprend le tissu hépatique ectopique.

15. Composition pour une utilisation selon l'une quelconque des revendications 1 à 13, dans laquelle l'au moins une cellule comprend la cellule rénale, le fragment de tissu rénal, ou la combinaison de ceux-ci, et le tissu ectopique comprend le tissu rénal ectopique.
